# EUROPÄISCHE PATENTSCHRIFT

(11) **EP 2 035 580 B1**
(45) Veröffentlichungstag und Bekanntmachung des Hinweises auf die Patenterteilung: **15.04.2015**
(21) Anmeldenummer: 07764586.9
(22) Anmeldetag: 06.06.2007
(51) Int. Cl.: C12Q 1/68

(54) **VERFAHREN ZUR FESTSTELLUNG VON PERITONITIS UND PNEUMONIE BEI SEPSISPATIENTEN**
METHOD FOR DETERMINING OF PERITONITIS AND PNEUMONIA IN PATIENTS WITH SEPSIS
PROCÉDÉ DE DÉTERMINATION DE PERITONITIS ET PNEUMONIE DANS PATIENTS ATTEINTS LA SEPTICÉMIE

(30) Priorität: 16.06.2006 DE 102006027842
(43) Veröffentlichungstag der Anmeldung: 18.03.2009
(73) Patentinhaber: Analytik Jena AG, 07745 Jena (DE)
(72) Erfinder: RUSSWURM, Stefan, 07743 Jena (DE); REINHART, Konrad, 07743 Jena (DE); BAUER, Michael, 07743 Jena (DE)
(74) Vertreter: Winter, Brandl, Fürniss, Hübner, Röss, Kaiser, Polte - Partnerschaft mbB
(86) Internationale Anmeldenummer: PCT/EP2007/005043
(87) Internationale Veröffentlichungsnummer: WO 2007/144105

(56) Entgegenhaltungen:
- EP-A- 1 270 740
- WO-A-2004/087949
- WO-A-2005/068655
- DE-A1-102004 049 897
- DE-A1-102005 013 013
- US-A1- 2005 214 820
- ROTH A. R. ET AL.,: "approach to the adult patient with fever of unknown origin" AM. FAMILY PHYSICIAN, Bd. 68, Nr. 11, 1. Dezember 2003 (2003-12-01), Seiten 2221-2228, XP002459138 in der Anmeldung erwähnt

## Beschreibung

Die vorliegende Erfindung betrifft die Verwendung von in vitro aus einer Patientenprobe erhaltenen Genexpressionsprofilen für die für die Feststellung der lokalen Entzündung eines Fiebers unklarer Genese gemäß Anspruch-1, ein Verfahren zur in vitro Messung derartiger Genexpressionsprofile gemäß Anspruch 14 sowie die Verwendung der Genexpressionsprofile und/oder von den hierfür verwendeten Sonden zur Bestimmung der Genaktivität oder den davon abgeleiteten Proteinprodukten zum Screening von Wirkstoffen gegen Fieber mit unklarer Genese und/oder Peritonitis und/oder Pneumonie und/oder zur Beurteilung der Therapieeffekte von Wirkstoffen gegen Fieber mit unklarer Genese und/oder Peritonitis und/oder Pneumonie gemäß Anspruch 30 sowie einen Kit nach Anspruch 33.

Fieber unklarer Genese (Fever of unknow origin, FUO) ist klinisch definiert als ein Fieber, bei dem die Temperatur über einen Zeitraum von mehr als 3 Wochen höher als 38,8°C ist, ohne dass nach einer einwöchigen Untersuchungszeit eine eindeutige Diagnose der Ursache vorliegt. In Abhängigkeit des Ursprungs wurden vier Klassen des FUO beschrieben: FUO klassischen, nosokomialen, immunschwachen oder HIV-bezogenen Ursprungs (1). FUO wurde auch als "eine eher bekannte Krankheit mit einem ungewöhnlichen Erscheinungsbild als eine seltene Störung" geschildert (2).

Es gibt weder eine Goldstandard-Methode oder einen Diagnosetest, keine veröffentlichten Richtlinien noch auf Evidenz-basierende Empfehlungen zur Diagnose von FUO (3). Bis heute ist die Diagnose von FUO eine Herausforderung und wird unter Zuhilfenahme der Patientengeschichte, von Biopsien (z.B. Leber, Schläfenarterie), chirurgischen und/ oder bildgebender Verfahren wie abdominale Computertomographie oder nukleare Bildverfahren (3) durchgeführt. All diese Methoden sind sehr kostspielig und wegen des Eingriffs (Biopsie, Chirurgie) unangenehm für den Patienten (1) Hinsichtlich der diagnostizierten Hauptursache können folgende 4 Untergruppen definiert werden: Infektion, bösartiger Tumor, autoimmune Störung und sonstige Ursachen, wobei die Infektion die meist verbreitete Ursache von FUO ist (1,4).

Bei Patienten mit postoperativem Fieber wird nur bei 10% eine Infektion dokumentiert (5). In den meisten Fälle ging die Temperatur des Patienten innerhalb von vier Tagen nach dem Eingriff zurück auf normal. Trotzdem entwickelten einige Patienten am oder nach dem fünften postoperativem Tag eine Infektion, von denen 12% an einer Lungenentzündung erkrankten (5). Ebenso wird von Pile und Kollegen erwähnt, das Fieber, welches zwei Tage nach dem Eingriff auftrat, mit hoher Wahrscheinlichkeit von einer Infektion ausgelöst wurde, z.B. eine Infektion der Harnwege und/ oder des inneren Unterleibs (Peritonitis), Lungenentzündung, eine durch einen intravenösen Katheder ausgelöste Infektion.

Als dem FUO zugrunde liegende lokale Entzündungszustände kommen verschiedene Formen in Frage, wie beispielsweise Peritonitis, Pneumonie, Harnwegsinfektionen oder Endokarditis (2). Im folgenden wird beispielhaft auf Peritonitis und Pneumonie als zugrunde liegender Entzündungszustand eines FUO eingegangen.

Lungenentzündung ist einer der schwersten Infektionskrankheiten auf der Intensivstation die dramatische Auswirkungen auf die Lebenserwartung des Patienten haben kann (6,7). Bei der Lungenentzündung oder Pneumonie handelt es sich um eine akute oder chronische Entzündung des Lungenparenchyms die meist durch eine Infektion mit Bakterien, Viren oder Pilzen verursacht wird. Für die klinische-Diagnostik wird zwischen der ambulant und nosokomial erworbenen Pneumonie unterschieden. In den USA werden 2-3 Mio. Fälle von ambulant erworbener Pneumonie festgestellt, in Deutschland wird von etwa 750.000 Fälle von ambulant erworbener Pneumonie in Deutschland ausgegangen (8). Insgesamt belaufen sich die Kosten für die Pneumoniebehandlung allein in den USA auf etwa 8 Mrd. US$.

Eine Pneumonie wird als nosokomial definiert, wenn die Pneumonie 48 Stunden nach Einlieferung des Patienten in das Krankenhaus diagnostiziert wird (9). Das größte Risiko zur Entwicklung einer nosokomial erworbenen Pneumonie bei Intensivpatienten entsteht durch den Einsatz von Beatmungsmaschinen. Aus diesem Grund hat sich für diese Art Pneumonie der Begriff Ventilator-assozierte Pneumonie (VAP) durchgesetzt (10). Die Sterblichkeit bei VAP-Patienten liegt bei 30% (10).

Wie Sauer et al. berichten, konnten bei der Betrachtung von durch chirurgische Eingriffe ausgelöste Infektionen nur 30% der Infektionen nachgewiesen werden, die von einzelnen Erregern ausgelöst wurden. Sauer berichtet, dass die häufigste Infektionsursache bei Lungenentzündung Candida (Hefe) war. Bei Patienten mit Lungenentzündung konnten Mischinfektionen mit mindestens zwei Erregern (47%), ein einzelner Erreger (24 %) oder keine Mikroben (29%) nachgewiesen werden. Die Bestimmung einer möglichen Infektion und der Resistenz basiert auf klassischen mikrobiologischen Kultivierungsmethoden und auf Resistenztests gegenüber Antibiotika (11) und unterliegt aus diesem Grund auch den Beschränkungen solcher Methoden (nicht-kultivierbare Bakterien, eine längere Verzögerungsphase aufgrund des Gabe von Antibiotika, etc.).

Eine Peritonitis ist eine lokale Infektion der Bauchfells hervorgerufen durch den Eintritt von Bakterien oder Pilzen in den Bachraum. Peritoneale mesotheliale Zellen (PMC) im muskulären Teil der Membran werden von intermesothelialen Lücken (Stomata) unterbrochen und ermöglichen so den Kontakt mit den Hohlräumen (Lakunen) im Lymphgefäß und den Austritt der Bakterien aus der Bauchhöhle (12). Nach Hall et al. erklärt die schnelle Beseitigung der Bakterien aus der Bauchhöhle die initiale septische Phase einer Peritonitis. Eine Infektion der Bauchhöhle wird durch drei verschiedene Mechanismen bewältigt: 1. Induktion einer Immunabwehr wie zum Beispiel die Freisetzung von Inflammationsmediatoren, 2. die Migration polymorphonuklearer Neutrophile und der Komplementkaskade und 3. die Herausbildung eines Abszesses.

Peritonitis involviert üblicherweise gemischte mikrobielle Populationen (12), nichtsdestotrotz variiert der Ausgang einer Peritonitis in Abhängigkeit des Erregers, der die Peritonitis verursacht hat (13). So beschreiben Troidle et al., dass Gramnegative Infektionen zu einer höheren Mortalität führen und dass diese Patienten mit einer höheren Wahrscheinlichkeit einen Krankenhausaufenthalt benötigten als bei Gram-positiven Erregern. Verglichen mit Gram-negativer Peritonitis (9%) kommt es bei Gram-positiver Peritonitis in 32% der Fälle zu einem Wiederauftreten der Infektion zu einem späteren Zeitpunkt mit demselben Erreger. Trotz vieler Publikationen, welche die Auswirkung der Erreger auf den Patienten darstellen (z.B. 12), stufen einige Autoren die Wintsreaktion auf eine Infektion als wichtiger als die Infektion selber ein (14). Diese aus Tiermodellen ermittelten Beurteilungen beruhten allerdings auf einem physiologischem Bewertungssystem und setzten keine genomischen oder proteomischen Experimente ein.

Neue molekularbiologische Methoden erlauben die Untersuchung der immunologischen Wirtsreaktion auf eine Infektion. So sind aus dem Stand der Technik verschiedene Methoden und Ergebnisse bekannt, die die differentielle Genaktivität als Antwort auf eine durch eine Infektion verursachte Erkrankung beschreiben (15-19).

Die grundsätzliche Verwendbarkeit von Genexpressionsprofilen, welche beispielsweise mittels der Microarray-Technik erhalten werden können, zur Diagnose von SIRS, generalisierten inflammatorischen Entzündungen, Sepsis und schwerer Sepsis ist in der PCT-Patentanmeldung der Anmelderin der vorliegenden Erfindung (20) oder (21), beschrieben, auf die hiermit vollinhaltlich Bezug genommen wird.

In der deutschen Patentanmeldung (22) werden erstmals Genaktiviätsmarker zur Unterscheidung zwischen infektiösem und nicht infektiösem Multiorganversagen gezeigt. Darin wird die Verwendung von 1297 verschiedene Genen für die in vitro Diagnose von Patienten; die an einem infektiösem bzw. nicht infektiösem Multiorganversagen erkrankt sind, beschrieben.

Ebenfalls konnten verschiedene organspezifische Studien zur differentiellen Genexpression hervorgerufen durch lokale Entzündungen gezeigt werden, wie beispielsweise durch Untersuchungen von Lungengewebe (19, 23-25) oder durch Untersuchungen der veränderten Genaktivität von Lebergwebe als Reaktion auf eine fäkale Peritontis (26). Diese Untersuchungen bezogen sich aber immer auf gewebespezifische Genaktivitätsänderungen und somit nicht zur Feststellung eines FUO durch Messung der Genaktivität in Körperflüssigkeiten geeignet.

In der Patentanmeldung (27) wird die Genexpression zur Einschätzung, des infektiösen bzw. nicht-infektiösen Zustandes der identifizierten Infektionsquelle und nicht zur Feststellung der Infektionsquelle verwendet. So wird beispielsweise bestimmt, ob eine Infektion im-Kniegelenk vorliegt, indem eine Biopsie durchgeführt wird und die in der Gelenkflüssigkeit enthaltenen Zellen analysiert werden. Diese Erfindung lehrt nicht die Untersuchung der differentiellen Genaktivität in Körperflüssigkeiten zur Feststellung der zugrunde liegenden lokalen Entzündung eines FUO.

In Reinhart et al. (28) sowie aus der noch nicht vorveröffentlichten deutschen Patentanmeldung (29) der Anmelderin dieser Erfindung (28) wurden aus Vollblut gewonnene Genexpressionsprofile von Patienten, bei denen SIRS bzw. Sepsis diagnostiziert waren, präsentiert. Die differentielle Genaktivität wurde benutzt um einzuschätzen, ob Genaktivitäts-Klassifikatoren zwischen infektiösen und nichtinfektiösen Entzündungserkrankungen differenzieren können. In dieser Studie wurden die experimentell ermittelten Genaktivitäts-Klassifikatoren anschließend mit den klinischen Parametern, die von den Patienten zur Verfügung standen, verglichen. Es konnte gezeigt werden, dass die identifizierten Genaktivitäts-Klassifikatoren eine gute Unterscheidungskraft zwischen infektiösen und nicht-infektiösen Zuständen besitzen, wenn die klinischen Daten auf eine Peritonitis als zugrundelegende lokale Entzündung verwiesen. Allerdings war Unterscheidungskraft zwischen infektiösen und nicht-infektiösen Zuständen geringer, wenn die klinischen Daten eine Ventilator-assozierte Pneumonie (VAP) anzeigten. Die in Reinart (2005) bzw. in Referenz 29 beschriebenen Genaktivitätsklassifikatoren erlauben somit eine Unterscheidung zwischen infektiösen und nicht-infektiösen Zuständen. Eine Möglichkeit zur Feststellung der zugrunde liegenden lokalen Entzündung eines FUO anhand von Genexpressionsprofilen wurde werde offenbart noch nahe gelegt.

Es besteht somit ein dringender Bedarf an Möglichkeiten zur in vitro Diagnose der zugrunde liegenden lokalen Entzündung bei einem Fieber unklarer Genese. Die Verfügbarkeit solcher in vitro Verfahren wird erheblich zu einer schnellen und für den Patienten wenig belastenden Diagnose eines FUO führen, ädequate therapeutische Maßnahmen erlauben sowie die Kosten der Behandlung signifikant reduzieren.

Ausgangspunkt für die in der vorliegenden Patentanmeldung offenbarten Erfindung ist die Erkenntnis, dass sich anhand von Genaktivitätsprofilen die zugrunde liegende lokalen Entzündung eines FUO bestimmen werden kann. Die Verwendung dieser Genaktivitäten ist mit den bisher zur Diagnose verwendeten klinischen Parametern nicht möglich, aber für die Einleitung einer spezialisierten intensivmedizinischen Therapie sehr bedeutungsvoll.

Der vorliegenden Erfindung liegt somit die Aufgabe zugrunde, durch die Verwendung von Genaktivitätsmarkern die Feststellung lokalen Entzündung eines Fiebers unklarer Genese zu ermöglichen.

Diese Aufgabe wird durch die Merkmale der Ansprüche 1-11 gelöst.

Die Verwendung von in vitro aus einer Patientenprobe erhaltenen Genexpressionsprofilen für die Feststellung der lokalen Entzündung eines Fiebers unklarer Genes ist beschrieben.

Die Genxpressionsprofile von wenigstens 2 Polynukleotiden, ausgewählt aus den SEQ-IDs No 1 bis 191 aufgenommen werden, die spezifisch für Peritonitis oder Pneumonie als lokalen Entzündung eines "Fiebers unklarer Genese" sind. Hierbei können die in ihrem Expressionsverhalten vergleichbaren Genaktivitäten der Polynukleotide mit den SEQ-IDs No 1 bis 191 zu diagnostischen Genaktivitätsclustern zusammengefasst werden.

Diese Genaktivitätscluster setzen sich wie folgt zusammen:

| | |
|---|---|
| Cluster 1: | SEQ-ID No.1 bis SEQ-ID No. 77 Peritonits spezifische Sequenzen mit signifikanter Genaktiviät (Tab. 3) |
| Cluster 2: | SEQ-ID No. 78 bis SEQ-ID No. 191 Pneumonie spezifische Sequenzen mit signifikanter Genaktiviät (Tab.3) |

Die Beschreibung umfasst ebenfalls Genexpressionsprofile von wenigstens 2 Polynukleotiden, ausgewählt aus den SEQ-ID No. 192 bis SEQ-ID No. 432 aufgenommen werden, die spezifisch für eine lokale Entzündung, nicht aber für Peritonitis oder Pneumonie, eines "Fiebers unklarer Genese" sind.

Die Erfindung schließt ebenfalls die Verwendung dieser Genexpressionsprofile als Ein- oder Ausschlußkriterium von Patienten mit "Fieber unklarer Genese" in klinische Studien ein.

Eine weitere Ausführungsform der Erfindung besteht in der Verwendung der in-vitro gewonnenen Genexpressionsprofile zur Erstellung von Genaktivitätsdaten für die elektronische Weiterverarbeitung. Diese Genaktivitätsdaten können zur Herstellung von Software für die Beschreibung der individuellen Prognose eines Patienten, für Diagnosezwecke und/oder Patientendatenmangementsysteme, eingesetzt werden.

Eine weitere Verwendung der in-vitro gewonnenen Genexpressionsprofile besteht in der Herstellung von klinischen Expertensystemen und/oder zur Modellierung von zelluläreren Signalübertragungswegen eingesetzt werden. Solche Modellierungsmethoden und/oder -programme sind beispielsweise Ingenuity (Fa. Ingenuity Systems), Panther (Applied Biosystems) oder andere dem Fachmann bekannte Verfahren.

In einer bevorzugten Ausführungsform ist dadurch gekennzeichnet, dass zur Erstellung des Genexpressionsprofiles ein spezifisches Gen und/oder Genfragment verwendet wird, welches ausgewählt wird aus der Gruppe bestehend aus SEQ-ID No. 1 bis SEQ-ID No. 191 sowie Genfragmenten davon mit wenigstens 20-2000 Nukleotiden.

Eine weitere Ausführungsform der Erfindung ist dadurch gekennzeichnet, dass die Genfragmente 20-200, vorzugsweise 20-80 Nukleotide umfassen.

Eine weitere Ausführungsform der Erfindung ist dadurch gekennzeichnet, dass die Genexpressionsprofile mittels Hybridisierungsverfahren, insbesondere solchen auf Microarrays oder real-time PCR basiert, ermittelt werden. Verfahren zur Hybridisierung sind dem Fachmann ausreichend bekannt.

Eine weitere Ausführungsform der Erfindung ist ein Verfahren, dadurch gekennzeichnet, dass zur in vitro Messung von Genexpressionsprofilen und/oder mindestens einem Genaktivitätscluster zur Feststellung der lokalen Entzündung eines Fiebers unklarer Genese, dadurch gekennzeichnet, dass man in Patienten die Genaktivität einer Mehrzahl von bestimmten, mit der Infektionsquelle in Zusammenhang stehenden Genen in einer Patientenprobe bestimmt.

Eine weitere Ausführungsform der Erfindung ist dadurch gekennzeichnet, dass zur in vitro Messung von Genexpressionsprofilen und/oder mindestens einem Genaktivitätscluster zur Feststellung von Peritonitis oder Pneumonie als Infektionsquelle eines Fiebers unklarer Genese, in Patienten die Genaktivität einer Mehrzahl von bestimmten, mit Peritonitis und Pneumonie als Infektionsquelle in Zusammenhang stehenden Gene in einer Patientenprobe bestimmt werden, wobei die für Peritonitis und Pneumonie der lokalen Entzündung spezifischen Gene und/oder Genfragmente ausgewählt werden aus der Gruppe bestehend aus: SEQ-ID No. 1 bis SEQ-ID No. 191 sowie Genfragmenten davon mit wenigstens 20-2000 Nukleotiden.

Eine weitere Ausführungsform der Erfindung ist dadurch gekennzeichnet, dass sich die spezifischen Sequenzen SEQ-ID No.1 bis SEQ-ID No. 191 aus folgenden diagnostischen Cluster zusammensetzen:

| | |
|---|---|
| Cluster 1: | SEQ-ID No.1 bis SEQ-ID No. 77 Peritonitis spezifische Sequenzen mit signifikanter Genaktiviät |
| Cluster 2: | SEQ-ID No. 78 bis SEQ-ID No. 191 Pneumonie spezifische Sequenzen mit signifikanter Genaktiviät |

Eine weitere Ausführungsform der Erfindung ist dadurch gekennzeichnet dass die Genfragmente 20-200, vorzugsweise 20-80 Nukleotide umfassen.

Eine weitere Ausführungsform der Erfindung ist dadurch gekennzeichnet dass wenigstens 4 bis 100 unterschiedliche Gene und Genfragmente verwendet werden.

Eine weitere Ausführungsform ist dadurch gekennzeichnet, dass wenigstens 200 unterschiedliche Gene und/oder Genfragmente verwendet werden.

Eine weitere Ausführungsform ist dadurch gekennzeichnet, dass wenigstens 200 bis 500 unterschiedliche Gene und/oder Genfragmente verwendet werden.

Eine weitere Ausführungsförm ist dadurch gekennzeichnet, dass wenigstens 500 bis 1000 unterschiedliche Gene und Genfragmente verwendet werden;

Eine weitere Ausführungsform ist dadurch gekennzeichnet, dass wenigstens 1000 bis 2000 unterschiedliche Gene und Genfragmente verwendet werden.

Eine weitere Ausführungsform ist dadurch gekennzeichnet, dass in Tabelle 3 und Tabelle 4 aufgelisteten Gene oder Genfragmente und/oder von deren RNA abgeleiteten Sequenzen ersetzt werden durch: synthetische Analoga, Aptamere, Spiegelmere sowie Peptido- und Morpholinonukleinsäuren.

Eine weitere Ausführungsform ist dadurch gekennzeichnet, dass die synthetischen Analoga der Gene 20-100, insbesondere ca. 70 Basenpaare umfassen.

Eine weitere Ausführungsform der Erfindung ist dadurch gekennzeichnet, dass die Genaktivitäten mittels Hybridisierungsverfahren bestimmt wird.

Eine weitere Ausführungsform der Erfindung ist dadurch gekennzeichnet, dass die Genaktivität mittels Microarrays bestimmt wird.

Eine weitere Ausführungsform der Erfindung ist dadurch gekennzeichnet, dass die Genaktivität durch Hybridisierungs-unabhängige Verfahren, insbesondere enzymatische und/oder chemische Hydrolyse und/oder Amplifikationsverfahren, vorzugsweise PCR, anschließende Quantifizierung der Nukleinsäuren und/oder von Derivaten und/oder Fragmenten derselben, bestimmt wird.

Die Erfindung ist dadurch gekennzeichnet, dass die Probe ausgewählt wird aus Blut.

In einer weitere Ausführungsform werden die in vitro aus einer Patientenprobe erhaltenen Genexpressionsprofilen und/oder von den hierfür verwendeten Sonden, welche ausgewählt werden aus der Gruppe bestehend aus SEQ-ID No. 1 bis SEQ-ID No. 191 sowie Genfragmenten davon mit wenigstens 20-2000 Nukleotiden, zur Bestimmung der Genaktivität oder den davon abgeleiteten Proteinprodukten zum Screening von Wirkstoffen gegen Fieber mit unklarer Genese und/oder Peritonitis und/oder Pneumonie und/oder zur Beurteilung der Therapieeffekte von Wirkstoffen gegen Fieber mit unklarer Genese und/oder Peritonitis und/oder Pneumonie verwendet.

Eine weitere Ausführungsform der Erfindung ist dadurch gekennzeichnet, dass hybridisierungsfähige synthetische Analoga der in den Tabellen 3 und 4 aufgelisteten Sonden verwendet werden.

Eine weitere Ausführungsform der Erfindung ist dadurch gekennzeichnet, dass die Genfragmente 20-200, vorzugsweise 20-80 Nukleotide umfassen.

Die Erfindung betrifft auch einen Kit, der eine Auswahl von Sequenzen die spezifisch für die Feststellung der lokalen Entzündung eines "Fiebers unklarer Genese"sind, und/oder Genfragmenten davon mit wenigstens 20-2000 Nukleotiden zur Bestimmung von Genexpressionsprofilen in vitro in einer Patientenprobe, für die Feststellung der Infektionsquelle und/oder der Infektionsursache eines Fiebers unklarer Genese, enthält.

Eine weitere Ausführungsform der Erfindung ist dadurch gekennzeichnet, dass der Kit eine Auswahl mit Sequenzen gemäß SEQ-ID No. 1 bis SEQ-ID No. 191 und/oder Genfragmenten davon mit wenigstens 20-2000 Nukleotiden zur Bestimmung von Genexpressionsprofilen in vitro in einer blüt Patientenprobe, für die Feststellung von Peritonitis und/oder Pneumonie bei Sepsispatienten, enthält.

### Ausführungsbeispiel

Untersuchung zur Erstellung von Genexpressionsprofilen zur Feststellung der lokalen Entzündung von Patienten, bei denen Fieber unklarer Genese (1,3) und eine schwere Infektion (30) diagnostiziert wurde.

### Messung der differentiellen Genexpression:

Zunächst wurde die differentielle Genexpression zwischen zwei Patientengruppen untersucht, von denen bekannt war, dass
i) die erste (teilweise geblindeten) Gruppe Patienten waren, die im Rahmen ihrer intensivmedizinischen Betreuung an einer schweren Infektion [Sepsis, klassifiziert nach 30] erkrankt waren und bei denen ein "Fieber unklarer Genese" diagnostiziert wurde (Patientengruppe 1). Von diese Patienten war nicht bekannt, welche die zugrunde liegende lokale Entzündung für das FUO war.
ii) die zweite Gruppe Patienten waren, die im Rahmen ihrer intensivmedizinischen Betreuung eine akute generalisierte Entzündung [SIRS, klassifiziert nach 30] mit Organversagen entwickelten, bei denen jedoch zu keinem Zeitpunkt ihrer intensivmedizinischen Betreuung eine Infektion nachgewiesen wurde (Patientengruppe 2).

Ausgewählte Charakteristika der zwei Patientengruppen sind in der Tabelle 1 dargestellt. Dabei werden Angaben zum Alter, Geschlecht, und dem SOFA-Score als Maß für die Funktion der Organsysteme gemacht. Gleichfalls sind die Plasmaproteinspiegel von Procalcitonin (PCT) und CRP sowie die Zahl der Leukozyten der Patienten angegeben.

Als Referenzproben dienten die totale RNA aus Zelllinien SIG-M5.

Alle Patientenproben wurden mit der Referenzprobe jeweils auf einem Microarray kohybridisiert.

**Tabelle 1: Daten der Patientengruppen 1 und 2**

| | **Patienten mit schwerer Infektion Patientengruppe 1** | **SIRS + OD Patientengruppe 2** |
|---|---|---|
| **Anzahl Patienten** | 39 | 37 |
| **Sterblichkeit** | 16 (41,0%) | 2° (5,4%) |
| **Geschlecht [M/W]** | 31/8 | 18/19 |
| **Alter [Jahre]** | 69 (11) | 75 (14) |
| **SOFA Score** | 9 (2.5) | 8* (2) |
| **Anzahl OD** | 3 (1) | 2 (1) |
| **PCT [ng/ml]** | 2.44 (3.20) [36] | 3.34 (4.13) [30] |
| **CRP [mg/l]** | 177 (124.4) [35] | 91.6* (90.13) [36] |
| **WBC [no/l]** | 14400 (9050) | 11900* (7400) |

| | | |
|---|---|---|
| median (IQR) *p < 0.05 (Wilcoxon-Rangsummen-Test) °p = 0.003 (Exakter Test von Fisher) | | |

### Experimentelle Beschreibung:

### Blutabnahme und RNA-Isolation

Das Vollblut der Patientengruppe 1 wurde postoperativ zum Zeitpunkt der Diagnose "Fieber unklarer Genese" von den Patienten mittels des PAXGene Kits gemäß den Vorgaben des Herstellers (Qiagen) abgenommen. Das Vollblut der Patientengruppe 2 wurde postoperativ mittels des PAXGene Kits gemäß den Vorgaben des Herstellers (Qiagen) abgenommen. Nach Abnahme des Vollblutes wurde die totale RNA der Proben unter Anwendung des PAXGene Blood RNA Kit gemäß den Vorgaben des Herstellers (Qiagen) isoliert.

### Zellkultivierung

Für die Zellkultivierung (Kontrollproben) wurden 19 Kryozellkulturen (SIGM5) (eingefroren in flüssigem Stickstoff) genutzt. Die Zellen wurden jeweils mit 2 ml Iscove's Medium (Biochrom AG) beimpft ergänzt mit 20% fetalen Kälber Serum (FCS). Die Zellkulturen wurden anschliessend für 24 Stunden bei 37° C unter 5% CO2 in 12-well Platten inkubiert. Danach wurde der Inhalt Von 18 Wells in 2 Teile mit jeweils dem gleichen Volumen geteilt, sodass schliesslich 3 Platten des gleichen Formats (insgesamt 36 Wells) zur Verfügung standen. Die Kultivierung wurde anschliessend für 24 Stunden unter den gleichen Bedingungen fortgeführt. Im Anschluss daran wurden die resultierenden Kulturen von 11 Wells jeder Platte vereint und zentrifugiert (1000 x g, 5 min, Raumtemperatur). Der Überstand wurde verworfen und das Zellpellet in 40 ml des o.g. Mediums gelöst. Diese 40 ml gelöste Zellen wurden in zwei 250 ml Kolben zu gleichen Teilen aufgeteilt und nach 48 Stunden Inkubation und Zugabe von 5 ml des o.g. Mediums wiederum inkubiert. Von den restlichen 2 ml der zwei verbleibendenden Platten wurden 80 µl in leere Wells der gleichen Platten gegeben, welche bereits vorher mit 1 ml des o.g. Mediums präpariert waren. Nach 48 Stunden Inkubation wurde nur eine der 12 Well-Platten wie folgt prozessiert: Aus jedem Well wurden 500 µl entnommen und vereint. Die daraus resultierenden 6 ml wurden in einen 250 ml Kolben gegeben, welcher ca. 10 ml frisches Medium enthielt. Dieses Gemisch wurde mit 1000 x g 5 Minuten bei Raumtemperatur zentrifugiert und in 10 ml des o.g. Mediums gelöst. Die anschliessende Zellzählung ergab folgendes Ergebnis: 1,5 x 107 Zellen pro ml, 10 ml Gesamtvolumen, Gesamtzahl der Zellen: 1,5 x 108. Da die Zellzahl noch nicht ausreichend war, wurden 2,5 ml des o.g. Zellsuspension in 30 ml des o.g. Mediums in einen 250 ml (75 cm²) Kolben gegeben (insgesamt 4 Kolben). Nach 72 Sunden Inkubationszeit wurden jeweils 20 ml frischen Mediums in die Kolben gegeben. Nach folgender 24-stündiger Inkubation erfolgte die Zellzählung wie oben beschrieben, die eine Gesamtzellzahl von 3,8 x 10⁸ Zellen ergab. Um die gewünschte Zellzahl von 2 x 106 Zellen zu errreichen wurden die Zellen in 47,5 ml des o.g. Mediums in 4 Kolben resuspendiert. Nach einer Inkubationszeit von 24 Stunden wurden die Zellen zentrifugiert und zweimal mit Phosahatpuffer ohne Ca²⁺ und Mg²⁺ (Biochrom AG) gewaschen.

Die Isolation der totalen RNA erfolgt mittels des NucleoSpin RNA L Kits (Machery&Nagel) entsprechend den Angaben des Herstellers. Die oben beschriebene Prozedur wurde wiederholt bis die erforderliche Zellzahl erreicht wurde. Dies war erforderlich, um die erforderliche Menge von 6 mg totale RNA zu erreichen, was etwa einer Effizienz von 600 µg RNA pro 108 Zellen entspricht.

### Reverse Transkription / Markierung / Hybridisierung

Nach Abnahme des Vollblutes wurde die totale RNA der Proben unter Verwendung des PAXGene Blood RNA Kits (PreAnalytiX) gemäss den Vorgaben des Herstellers isoliert und auf ihre Qualität geprüft. Von jeder Probe wurden 10 µg totale RNA aliquotiert und zusammen mit 10 µg total RNA aus SIGM5-Zellen als Referenz-RNA zu komplementärer DNA (cDNA) mit der reversen Transkriptase Superscript II (Invitrogen) umgeschrieben und die RNA anschließend durch alkalische Hydrolyse aus dem Ansatz entfernt. Im Reaktionsansatz wurde ein Teil des dTTP durch Aminoallyl-dUTP (AA-dUTP) ersetzt, um später die Kopplung des Fluoreszenzfarbstoffes an die cDNA zu ermöglichen.

Nach der Aufreinigung des Reaktionsansatzes wurden die cDNA der Proben und Kontrollen mit den Fluoreszenzfarbstoffen Alexa 647 und Alexa 555 kovalent markiert und auf einem Microarray der Firma SIRS-Lab hybridisiert. Auf dem verwendeten Microarray befinden sich 5308 immobilisierte Polynukleotide mit einer Länge von 55 - 70 Basenpaaren, die jeweils ein humanes Gen repräsentieren und Kontrollspots zur Qualitätssicherung. Ein Microarray unterteilt sich in 28 Subarrays mit einem Raster von 15x15 Spots.

Die Hybridisierung und das anschliessende Waschen bzw. Trocknen wurde in der Hybridisierungsstation HS 400 (Tecan) nach Angaben des Herstellers über 10,5 Stunden bei 42 °C durchgeführt. Die verwendete Hybridisierungslösung besteht aus den jeweiligen gelabelten cDNA-Proben, 3,5x SSC (1x SSC enthält 150 mM Natriumchlorid und 15 mM Natriumcitrat), 0,3% Natriumdodecylsulfat (V/V) 25% Formamid (V/V) und je 0,8 µg µl-1 cot-1 DNA, Hefe t-RNA und poly-A RNA. Das anschliessende Waschen der Mikroarrays wurde mit nachfolgendem Programm bei Raumtemperatur in durchgeführt: je 90 Sekunden spülen mit Waschpuffer 1 (2x SSC, 0,03% Natriumdodecylsulfat), mit Waschpuffer 2 (1x SSC) und abschließend mit Waschpuffer 3 (0,2x SSC). Danach wurden die Mikroarrays unter einem Stickstoffstrom mit einem Druck von 2,5 bar bei 30 °C über 150 Sekunden getrocknet.

Nach der Hybridisierung wurden die Hybridisierungssignale der Microarrays mit einem GenePix 4000B Scanner (Axon) ausgelesen und die Expressionsverhältnisse der differenziert exprimierten Gene mit der Software GenePix Pro 4.0 (Axon) bestimmt.

### Auswertung:

Für die Auswertung wurde die mittlere Intensität eines Spots als der Medianwert der zugehörigen Spotpixel bestimmt.

### Korrektur systematischer Fehler:

Die Korrektur systematischer Fehler erfolgte nach dem Ansatz von Huber et al. [31]. Dabei wurden der Additive und der multiplikative Bias innerhalb eines Microarrays aus 70% der vorhandenen Genproben geschätzt. Für alle weiteren Berechnungen wurden die Signale mittels arcus sinus hyperbolicus transformiert.

Für die Analyse wurden die normalisierten und transformierten relativen Verhältnisse der Signale der Patientenproben gegen die allgemeine Kontrolle berechnet. D.h. für das j-te Gen des n-ten Patienten ergab die Berechnung den Wert Gj,n = arcsinh(Scy5(j,n)) - arcsinh(Scy3(j,n)), wobei [SCy3(j,n), SCy5(j,n)] das zugehörige Signalpaar bezeichnet. War für einen Patienten ein Spot nicht auswertbar (z.B. Fleck auf dem gescannten Bild), so wurde der zugehörige Wert als nicht vorhanden (,missing value') gekennzeichnet.

### Statistischer Vergleich:

Für den Vergleich wurde der zweiseitige Zweistichproben Student-Test pro Gen verwendet. Beide Stichproben-enthielten die Werte der Patientengruppen. Für die Auswahl der differenziert exprimierten Gene wurde der zugehörige p-Wert bewertet. Für die Gruppe der ausgewählten Gene war der zugehörige p-Wert kleiner als 0.05.

Die in den Tabellen 3 und 4 aufgeführten sind in dem dieser Anmeldung angefügten Sequenzprotokol im Einzelnen jeweils einer Sequenz ID (Sequenz ID: 1 bis zur Sequenz ID: 432) zugeordnet.

Die ermittelten Genaktivitäten aus Tabellen und 3 und 4 können somit zur Unterscheidung von infektiösen und nicht-infektiösen Zuständen verwendet werden. Diese Ergebnisse bestätigen die Verfahren und Ergebnisse aus dem Stand der Technik, wie beispielsweise in (20-22) gezeigt.

### Entblindung der Patientengruppe 1 und Korrelation mit den ermittelten Genaktivitäten der Tabelle 3 und 4.

Aus der Entblindung der Patientengruppe 1 ergab sich, das diese Patientengruppe aus zwei Untergruppen bestand:
1) Patienten, bei denen ein FUO und eine schwere Infektion diagnostiziert wurde und bei denen die Follow-up Diagnose eine Peritonitis als zugrunde liegende lokale Entzündung identifiziert wurde (Patientengruppe 1 a).
2) Patienten, bei denen ein FUO und eine schwere Infektion diagnostiziert wurde und bei denen die Follow-up Diagnose eine Pneumonie als zugrunde liegende lokale Entzündung identifiziert wurde (Patientengruppe 1 b).

Ausgewählte Charakteristika der zwei Patientengruppen 1a und 1 b nach der Follow-up Diagnose sind in der Tabelle 2 dargestellt.

**Tabelle 2: Daten der Patientengruppen 1a und 1b**

| | **Patientengruppe 1a** | **Patientengruppe 1b** |
|---|---|---|
| **Anzahl Patienten** | 15 | 24 |
| **Sterblichkeit** | 9 (60%) | 7 (29,2 %) |
| **Geschlecht [M/W]** | 11/4 | 20/4 |
| **Alter [Jahre]** | 66 (9) | 70 (13) |
| **SOFA Score** | 9 (2.5) | 9 (2.25) |
| **Anzahl OD** | 3 (0) | 2.5 (1) |
| **PCT [ng/ml]** | 6.05 (24.3) [13] | 1.46* (1.98) [23] |
| **CRP [mg/l]** | 146 (87.5) | 206 (95.75) [20] |
| **WBC [no/l]** | 14400 (11000) | 14650 (6875) |
| **Lokale Entzündung** | Peritonitis | Pneumonie |

Zur Feststellung der einem FUO zugrunde liegendem lokalen Entzündung von Patienten, wurden die ermittelten Genaktivitäten aus Tabelle 3 und 4 statistisch nach signifikanten Genaktivitätsclustern klassifiziert, die innerhalb der Patientengruppen 1a und 1b eine ähnliche Aktivität aufwiesen. Dabei wurde überraschend festgestellt, dass sich ausgehend von allen gemessenen Genaktivitäten eine Klassifizierung der Genaktivitäten in drei Cluster ergeben hat.:

| | |
|---|---|
| Cluster 1: | Für Peritonitis wurde ein Cluster an spezifischen Sequenzen mit signifikanter Genaktivität entsprechend den SEQ-ID No. 1 bis SEQ-ID No. 77 ermittelt, die Bestandteil des angefügten Sequenzprotokolls sind. |
| Cluster 2: | Für Pneumonie wurde ein Cluster an spezifischen Sequenzen mit signifikanter Genaktivität entsprechend den SEQ-ID No. 78 bis SEQ-ID No. 191 ermittelt, die Bestandteil des angefügten Sequenzprotokolls sind. |
| Cluster 3: | Gemeinsamer Set an Sequenzen mit ähnlicher signifikanter Genaktivität bei Patienten mit schweren Infektionen, die spezifisch für eine lokale Entzündung, nicht aber für Peritonitis oder Pneumonie, eines "Fiebers unklarer Genese"sind. entsprechend den SEQ-ID No. 192 bis SEQ-ID No. 432 die Bestandteil des angefügten Sequenzprotokolls sind. |

Die drei Genaktivitätscluster sind in Tabelle 3 (cluster 1 und 2) und 4 (cluster 3) dargestellt.

**Tabelle 3: Genaktivitätscluster 1 und 2 zur Feststellung von Peritonitis (Cluster 1) oder Pneumonie (Cluster 2) als lokalen Entzündung eines FUO**

| | | **Normalisierte und transformierte Expressionssignale** | | | | | |
|---|---|---|---|---|---|---|---|
| **GenBank Accession Nummer** | **p value** | **mean Patientengruppe 2** | **mean Patientengruppe 1a** | **UniGene** | **Cluster** | **Untergruppe** | **SeqID** |
| AA029887 | 0,0003 | -0,145 | 0,174 | | 1 | Peritonitis | 1 |
| AA149226 | 0,0028 | -0,248 | 0,298 | Hs.494192 | 1 | Peritonitis | 2 |
| AA398757 | 0,0000 | -0,665 | 0,798 | Hs.634201 | 1 | Peritonitis | 3 |
| AA402274 | 0,0110 | -0,216 | 0,259 | Hs 567266 | 1 | Peritonitis | 4 |
| AA419092 | 0,0046 | 0,152 | -0,182 | Hs.122575 | 1 | Peritonitis | 5 |
| AA435854 | 0,0000 | 0,197 | -0,236 | | 1 | Peritonitis | 6 |
| AA441793 | 0,0081 | -0,159 | 0,191 | Hs.132753 | 1 | Peritonitis | 7 |
| AA458827 | 0,0002 | -0,42 | 0,503 | Hs.500546 | 1 | Peritonitis | 8 |
| AA479285 | 0,0296 | -0,094 | 0,113 | Hs.536450 | 1 | Peritonitis | 9 |
| AA490815 | 0,0073 | 0,144 | -0,173 | Hs.558393 | 1 | Peritonitis | 10 |
| AA620762 | 0,0272 | -0,14 | 0,168 | Hs.371845 | 1 | Peritonitis | 11 |
| AA629051 | 0,0017 | 0,13 | -0,156 | | 1 | Peritonitis | 12 |
| AA693514 | 0,0224 | 0,09 | -0,107 | Hs.134229 | 1 | Peritonitis | 13 |
| AA708806 | 0,0014 | -0,129 | 0,154 | Hs.596038 | 1 | Peritonitis | 14 |
| AA731679 | 0,0415 | -0,235 | 0,282 | Hs.128619 | 1 | Peritonitis | 15 |
| AI057616 | 0,0207 | -0,102 | 0,123 | Hs.83761 | 1 | Peritonitis | 16 |
| AI086719 | 0.0347 | 0,105 | -0,126 | Hs.127657 | 1 | Peritonitis | 17 |
| AI128170 | 0,0432 | 0,111 | -0,133 | Hs.592034 | 1 | Peritonitis | 18 |
| AI150418 | 0,0353 | 0,111 | -0,133 | Hs.545647 | 1 | Peritonitis | 19 |
| AI218498 | 0,0480 | 0,113 | -0,135 | Hs.585282 | 1 | Peritonitis | 20 |
| AI221860 | 0,0304 | -0,229 | 0,275 | Hs.208353 | 1 | Peritonitis | 21 |
| AI241294 | 0,0484 | 0,125 | -0,15 | Hs.308641 | 1 | Peritonitis | 22 |
| AI282924 | 0,0001 | -0,166 | 0,199 | Hs.591290 | 1 | Peritonitis | 23 |
| AI291041 | 0,0003 | 0,215 | -0,258 | | 1 | Peritonitis | 24 |
| AI374599 | 0,0015 | 0,132 | -0,158 | Hs.128060 | 1 | Peritonitis | 25 |
| AI418437 | 0,0066 | -0,264 | 0,317 | Hs.534383 | 1 | Peritonitis | 26 |
| AI420865 | 0,0035 | -0,148 | 0,178 | Hs.541901 | 1 | Peritonitis | 27 |
| AI499146 | 0,0126 | -0,147 | 0,176 | | 1 | Peritonitis | 28 |
| AI520932 | 0,0211 | -0,111 | 0,133 | Hs.516707 | 1 | Peritonitis | 29 |
| AI627453 | 0,0401 | 0,104 | -0,125 | Hs.370510 | 1 | Peritonitis | 30 |
| AI634473 | 0,0158 | 0,107 | -0,128 | Hs.603284 | 1 | Peritonitis | 31 |
| AI696984 | 0,0069 | -0,121 | 0,145 | Hs.262907 | 1 | Peritonitis | 32 |
| AI732971 | 0,0169 | 0,179 | -0,214 | Hs.559775 | 1 | Peritonitis | 33 |
| AI912592. | 0,0005 | -0,148 | 0,177 | Hs.86538 | 1 | Peritonitis | 34 |
| BC004983 | 0,0486 | -0,103 | 0,124 | Hs.81328 | 1 | Peritonitis | 35 |
| BC032713 | 0,0047 | -0,131 | 0,157 | Hs.87968 | 1 | Peritonitis | 36 |
| CARD10 | 0,0003 | -0,154 | 0,184 | Hs.57973 | 1 | Peritonitis | 37 |
| CCL15.2 | 0,0184 | -0,096 | 0,115 | Hs.272493 | 1 | Peritonitis | 38 |
| CCL26 | 0,0259 | 0,127 | -0,153 | Hs.131342 | 1 | Peritonitis | 39 |
| CCL27 | 0,0082 | 0,129 | -0,154 | Hs.459590 | 1 | Peritonitis | 40 |
| CR1 | 0,0001 | -0,167 | 0,2 | Hs.334019 | 1 | Peritonitis | 41 |
| DNAJB2 | 0,0000 | -0,226 | 0,271 | Hs.77768 | 1 | Peritonitis | 42 |
| FADD | 0,0004 | -0,153 | 0,184 | Hs.86131 | 1 | Peritonitis | 43 |
| GH1.1 | 0,0033 | -0,268 | 0,322 | Hs.406754 | 1 | Peritonitis | 44 |
| H05223 | 0,0000 | -0,175 | 0,21 | Hs.124638 | 1 | Peritonitis | 45 |
| H11661 | 0,0000 | -0,223 | 0,267 | Hs.504091 | 1 | Peritonitis | 46 |
| H91663 | 0,0009 | -0,151 | 0,181 | Hs.208052 | 1 | Peritonitis | 47 |
| IF | 0,0084 | -0,158 | 0,19 | Hs.312485 | 1 | Peritonitis | 48 |
| IL21R | 0,0065 | -0,179 | 0,214 | Hs.210546 | 1 | Peritonitis | 49 |
| IL2RB | 0,0223 | 0,341 | -0,409 | Hs.474787 | 1 | Peritonitis | 50 |
| KBRAS2 | 0,0000 | -0,204 | 0,244 | Hs.632252 | 1 | Peritonitis | 51 |
| MAP2K2 | 0,0000 | -0,195 | 0,235 | Hs.465627 | 1 | Peritonitis | 52 |
| N64541 | 0,0088 | 0,172 | -0,207 | Hs.597199 | 1 | Peritonitis | 53 |
| NM_004710 | 0,0388 | -0,088 | 0,105 | Hs.464210 | 1 | peritonitis | 54 |
| NM-001774 | 0,0002 | -0,192 | 0,23 | Hs.166556 | 1 | Peritonitis | 55 |
| NM-002649 | 0,0001 | -0,177 | 0,212 | Hs.602240 | 1 | Peritonitis | 56 |
| NM-006058 | 0,0018 | -0,208 | 0,25 | Hs.543850 | 1 | Peritonitis | 57 |
| NM-006732 | 0,0046 | -0,255 | 0, 307 | Hs.590958 | 1 | Peritonitis | 58 |
| NM-014339 | 0,0019 | -0,146 | 0,175 | Hs.129751 | 1 | Peritonitis | 59 |
| NM-139276 | 0,0008 | -0,143 | 0,172 | Hs.463059 | 1 | Peritonitis | 60 |
| NOX2 | 0,0008 | -0,154 | 0,184 | Hs,292356 | 1 | Peritonitis | 61 |
| PLAT.2 | 0,0116 | -0,154 | 0,184 | Hs.491582 | 1 | Peritonitis | 62 |
| PPARD | 0,0000 | -0,179 | 0,215 | Hs.485196 | 1 | Peritonitis | 63 |
| R26118 | 0,0059 | 0,184 | -0,22 | Hs.594374 | 1 | Peritonitis | 64 |
| R36650 | 0,0052 | -0,126 | 0,151 | Hs.591522 | 1 | Peritonitis | 65 |
| R43074 | 0,0480 | 0,08 | -0,096 | Hs.298851 | 1 | Peritonitis | 66 |
| R53961 | 0,0118 | -0,106 | 0,127 | Hs.124128 | 1 | Peritonitis | 67 |
| R96155 | 0,0026 | 0,131 | -0,157 | | 1 | Peritonitis | 68 |
| TNFRSF6B.1 | 0,0045 | -0,122 | 0,146 | Hs.434878 | 1 | Peritonitis | 69 |
| TRIAD3 | 0,0039 | -0,143 | 0,172 | Hs.487458 | 1 | Peritonitis | 70 |
| TUCAN | 0,0001 | -0,153 | 0,183 | Hs.446146 | 1 | Peritonitis | 71 |
| W32272 | 0,0025 | 0,146 | -0,176 | Hs.594426 | 1 | Peritonitis | 72 |
| W85706 | 0,0000 | -0,214 | 0,257 | Hs.458973 | 1 | Peritonitis | 73 |
| XM-001687 | 0,0027 | -0,155 | 0,186 | Hs.77867 | 1 | Peritonitis | 74 |
| XM-006800 | 0,0000 | -0,235 | 0,282 | Hs.591043 | 1 | Peritonitis | 75 |
| XM-032902 | 0,0063 | 0,191 | -0,23 | Hs.593754 | 1 | Peritonitis | 76 |
| XM-036966 | 0,0190 | -0,097 | 0,116 | Hs.431850 | 1 | Peritonitis | 77 |

| **GenBank Accession Nummer** | **p value** | **mean Patientengruppe 2** | **mean Patientengruppe 1b** | **UniGene** | **Cluster** | **Untergruppe** | **SeqID** |
|---|---|---|---|---|---|---|---|
| AA017263 | 0,0099 | 0,16 | -0,127 | Hs.156727 | 2 | Pneumonie | 78 |
| AA017301 | 0,0179 | 0,139 | -0,11 | Hs.85863 | 2 | Pneumonie | 79 |
| AA031731 | 0,0015 | -0,358 | 0,283 | Hs.238964 | 2 | Pneumonie | 80 |
| AA398760 | 0,0275 | 0,213 | -0,168 | Hs.570638 | 2 | Pneumonie | 81 |
| AA400434 | 0,0088 | 0,137 | -0,108 | Hs.563200 | 2 | Pneumonie | 82 |
| AA400470 | 0,0418 | 0,146 | -0,115 | Hs.97805 | 2 | Pneumonie | 83 |
| AA402483 | 0,0138 | 0,169 | -0,134 | Hs.97313 | 2 | Pneumonie | 84 |
| AA417980 | 0,0004 | -0.281 | 0,223 | Hs.479226 | 2 | Pneumonie | 85 |
| AA428463 | 0,0195 | -0,186 | 0,147 | Hs.372739 | 2 | Pneumonie | 86 |
| AA436250 | 0,0019 | -0,361 | 0,286 | Hs.490203 | 2 | Pneumonie | 87 |
| AA452113 | 0,0054 | 0,144 | -0,114 | Hs.500643 | 2 | Pneumonie | 88 |
| AA458912 | 0,0330 | 0,167 | -0,132 | Hs.281898 | 2 | Pneumonie | 89 |
| AA459648 | 0,0086 | 0,154 | -0,122 | | 2 | Pneumonie | 90 |
| AA478611 | 0,0132 | -0,221 | 0,175 | Hs.105616 | 2 | Pneumonie | 91 |
| AA479727 | 0,0239 | 0,128 | -0,101 | Hs.23158 | 2 | Pneumonie | 92 |
| AA514450 | 0,0107 | 0,141 | -0,112 | | 2 | Pneumonie | 93 |
| AA541644 | 0,0000 | -0,218 | 0,173 | Hs.232165 | 2 | Pneumonie | 94 |
| AA548307 | 0,0234 | 0,137 | -0,108 | Hs.399800 | 2 | Pneumonie | 95 |
| AA626313 | 0,0137 | 0,142 | -0,113 | Hs.116150 | 2 | Pneumonie | 96 |
| AA628539 | 0,0280 | -0,132 | 0,105 | Hs.371001 | 2 | Pneumonie | 97 |
| AA699706 | 0,0043 | -0,268 | 0,212 | Hs.464779 | 2 | Pneumonie | 98 |
| AA844053 | 0,0402 | 0,119 | -0,094 | Hs.535257 | 2 | Pneumonie | 99 |
| AA887470 | 0,0423 | -0,12 | 0, 095 | Hs.531081 | 2 | Pneumonie | 100 |
| AA897543 | 0,0122 | 0,235 | -0,186 | Hs.148217 | 2 | Pneumonie | 101 |
| AA906116 | 0, 0083 | 0,264 | -0,209 | Hs.521545 | 2 | Pneumonie | 102 |
| AA910923 | 0,0030 | 0,194 | -0,153 | Hs.191164 | 2 | Pneumonie | 103 |
| AA935135 | 0,0222 | 0,148 | -0,117 | Hs.585129 | 2 | Pneumonie | 104 |
| AA969039 | 0,0051 | 0,152 | -0,121 | Hs.544636 | 2 | Pneumonie | 105 |
| AA992540 | 0,0002 | 0,258 | -0,204 | Hs.491869 | 2 | Pneumonie | 106 |
| ADRA2A | 0,0391 | -0,116 | 0,092 | Hs.249159 | 2 | Pneumonie | 107 |
| AF077011 | 0,0003 | 0,17 | -0,135 | Hs.459095 | 2 | Pneumonie | 108 |
| AI005466 | 0,0005 | 0,175 | -0,139 | Hs.602706 | 2 | Pneumonie | 109 |
| AI023336 | 0,0002 | 0,217 | -0,172 | Hs.370267 | 2 | Pneumonie | 110 |
| AI140065 | 0.0091 | -0.25 | 0,198 | Hs.146594 | 2 | Pneumonie | 111 |
| AI142427 | 0,0101 | -0,219 | 0,174 | Hs.300684 | 2 | Pneumonie | 112 |
| AI150305 | 0,0231 | 0.202 | -0.16 | Hs.128031 | 2 | Pneumonie | 113 |
| AI160757 | 0,0119 | 0,225 | -0,178 | Hs.408960 | 2 | Pneumonie | 114 |
| AI191762 | 0,0381 | 0,206 | -0,163 | Hs.495918 | 2 | Pneumonie | 115 |
| AI264774 | 0,0041 | 0,17 | -0,135 | Hs.514242 | 2 | Pneumonie | 116 |
| AI272798 | 0,0478 | -0,103 | 0,082 | Hs.479808 | 2 | Pneumonie | 117 |
| AI285411 | 0,0306 | -0,142 | 0,113 | Hs.635265 | 2 | Pneumonie | 118 |
| AI375046 | 0,0005 | 0,238 | -0,189 | | 2 | Pneumonie | 119 |
| AI421397 | 0,0391 | -0,117 | 0,093 | Hs.507025 | 2 | Pneumonie | 120 |
| AI492528 | 0,0268 | -0,158 | 0,125 | | 2 | Pneumonie | 121 |
| AI554942 | 0,0448 | 0,228 | -0,181 | Hs.570675 | 2 | Pneumonie | 122 |
| AI568793 | 0,0058 | -0,322 | 0,255 | Hs.368944 | 2 | Pneumonie | 123 |
| AI625724 | 0,0046 | -0,225 | 0,178 | Hs.185597 | 2 | Pneumonie | 124 |
| AI635650 | 0,0212 | 0,131 | -0,104 | | 2 | Pneumonie | 125 |
| AI654928 | 0,0000 | 0,272 | -0,216 | Hs.196133 | 2 | Pneumonie | 126 |
| AI685048 | 0,0004 | -0,165 | 0,131 | Hs.369785 | 2 | Pneumonie | 127 |
| AI700169 | 0,0010 | -0,334 | 0,264 | Hs.584910 | 2 | Pneumonie | 128 |
| AI745409 | 0,0032 | -0,265 | 0,21 | Hs.204924 | 2 | Pneumonie | 129 |
| AI798514 | 0,0314 | 0,143 | -0,113 | Hs.632218 | 2 | Pneumonie | 130 |
| AI799683 | 0,0047 | -0,149 | 0,118 | Hs.592083 | 2 | Pneumonie | 131 |
| AI799767 | 0,0323 | 0,22 | -0,175 | Hs.209226 | 2 | Pneumonie | 132 |
| AI801504 | 0,0108 | -0,135 | 0,107 | Hs.16064 | 2 | Pneumonie | 133 |
| AI808903 | 0,0240 | -0,15 | 0,118 | Hs.519855 | 2 | Pneumonie | 134 |
| AI811774 | 0,0115 | -0,148 | 0,117 | | 2 | Pneumonie | 135 |
| AI861979 | 0,0148 | 0,143 | -0,114 | Hs.469134 | 2 | Pneumonie | 136 |
| AI866656 | 0,0007 | 0,164 | -0,13 | Hs.211814 | 2 | Pneumonie | 137 |
| BC001604 | 0,0001 | -0,214 | 0,169 | Hs.182014 | 2 | Pneumonie | 138 |
| C2 | 0,0016 | 0,165 | -0,13 | Hs.408903 | 2 | Pneumonie | 139 |
| H02254 | 0,0280 | 0,245 | -0,194 | Hs.632489 | 2 | Pneumonie | 140 |
| H05436 | 0,0000 | 0,241 | -0,191 | Hs.11110 | 2 | Pneumonie | 141 |
| H11068 | 0,0045 | -0,174 | 0,138 | Hs.317243 | 2 | Pneumonie | 142 |
| H22946 | 0,0015 | 0,171 | -0,135 | Hs.534590 | 2 | Pneumonie | 143 |
| H38159 | 0,0026 | 0,211 | -0,167 | Hs.162996 | 2 | Pneumonie | 144 |
| H50201 | 0,0430 | -0,17 | 0,135 | Hs.21413 | 2 | Pneumonie | 145 |
| H73724 | 0,0002 | 0,211 | -0,167 | Hs.119882 | 2 | Pneumonie | 146 |
| H83996 | 0,0020 | 0,147 | -0,916 | Hs.153458 | 2 | Pneumonie | 147 |
| ICAM | 40,0113 | 0,118 | -0,093 | Hs.631609 | 2 | Pneumonie | 148 |
| IL18mRNA | 0,0001 | -0,182 | 0,144 | Hs.83077 | 2 | Pneumonie | 149 |
| IL26mRNA | 0,0293 | 0,108 | -0,085 | Hs.272350 | 2 | Pneumonie | 150 |
| LTBP4 | 0,0499 | -0,161 | 0,128 | Hs.466766 | 2 | Pneumonie | 151 |
| LTBR | 0,0191 | -0,164 | 0,13 | Hs.1116 | 2 | Pneumonie | 152 |
| M37435 | 0,0488 | -0,109 | 0,087 | Hs.173894 | 2 | Pneumonie | 153 |
| MAP4K4 | 0,0192 | -0,13 | 0,103 | Hs.431550 | 2 | Pneumonie | 154 |
| MAPK11.1 | 0,0268 | 0,116 | -0,092 | Hs.57732 | 2 | Pneumonie | 155 |
| MAPK7 | 0,0195 | -0,233 | 0,184 | Hs.150136 | 2 | Pneumonie | 156 |
| N29761 | 0,0156 | 0,552 | -0,437 | Hs.9315 | 2 | Pneumonie | 157 |
| N49848 | 0,0384 | 0,213 | -0,168 | Hs.104091 | 2 | Pneumonie | 158 |
| N55249 | 0,0426 | -0,185 | 0,147 | Hs.143347 | 2 | Pneumonie | 159 |
| N64649 | 0,0181 | 0,166 | -0,131 | Hs.102402 | 2 | Pneumonie | 160 |
| N69363 | 0,0284 | 0,158 | -0,125 | Hs.594444 | 2 | Pneumonie | 161 |
| N70546 | 0,0071 | -0,253 | 0,2 | Hs.155040 | 2 | Pneumonie | 162 |
| N80868 | 0,0128 | 0,143 | -0,113 | Hs.211426 | 2 | Pneumonie | 163 |
| NFKBIL2 | 0,0003 | -0,184 | 0,146 | Hs.459376 | 2 | Pneumonie | 164 |
| NM-001295 | 0,0001 | -0,189 | 0,149 | Hs.301921 | 2 | Pneumonie | 165 |
| NM-057158 | 0,0214 | 0,169 | -0,134 | Hs.417962 | 2 | Pneumonie | 166 |
| NOX1.2 | 0,0265 | 0,239 | -0,189 | Hs.592227 | 2 | Pneumonie | 167 |
| R00206 | 0,0028 | 0,199 | -0,158 | Hs.533551 | 2 | Pneumonie | 168 |
| R09463 | 0,0145 | 0,176 | -0,139 | Hs.344165 | 2 | Pneumonie | 169 |
| R16722 | 0,0334 | 0,221 | -0,175 | Hs.124246 | 2 | Pneumonie | 170 |
| R41771 | 0,0013 | 0,153 | -0,121 | Hs.591601 | 2 | Pneumonie | 171 |
| R42593 | 0,0207 | 0,154 | -0,122 | Hs.80395 | 2 | Pneumonie | 172 |
| R42778 | 0,0077 | 0,215 | -0,17 | Hs.553877 | 2 | Pneumonie | 173 |
| R43910 | 0,0009 | 0,398 | -0,315 | Hs.586760 | 2 | Pneumonie | 174 |
| R46801 | 0,0070 | -0,172 | 0,136 | Hs.343664 | 2 | Pneumonie | 175 |
| R49244 | 0,0078 | 0,125 | -0,099 | Hs.443258 | 2 | Pneumonie | 176 |
| R50755 | 0,0000 | 0,192 | -0,152 | Hs.633191 | 2 | Pneumonie | 177 |
| R60898 | 0,0032 | 0,136 | -0,108 | Hs.568242 | 2 | Pneumonie | 178 |
| R62926 | 0,0257 | 0,169 | -0,134 | Hs.285193 | 2 | Pneumonie | 179 |
| R79239 | 0,0087 | 0,18 | -0,143 | Hs.530588 | 2 | Pneumonie | 180 |
| R80259 | 0,0020 | 0,16 | -0,126 | Hs.595477 | 2 | Pneumonie | 181 |
| TGFB1 | 0,0136 | -0,122 | 0,097 | Hs.155218 | 2 | Pneumonie | 182 |
| W88496 | 0,0318 | 0,214 | -0,17 | Hs.314413 | 2 | Pneumonie | 183 |
| X57817 | 0,0119 | -0,639 | 0,506 | Hs.561078 | 2 | Pneumonie | 184 |
| XM-006953 | 0,0341 | 0,208 | -0,165 | Hs.355307 | 2 | Pneumonie | 185 |
| XM-028642 | 0,0149 | -0,175 | 0,139 | H.505654 | 2 | Pneumonie | 186 |
| XM-033972 | 0,0496 | -0,121 | 0,096 | Hs.492740 | 2 | Pneumonie | 187 |
| XM-034166 | 0,0028 | 0,149 | -0,118 | Hs.462525 | 2 | Pneumonie | 188 |
| XM-041844 | 0,0194 | 0,159 | -0,126 | Hs.36 | 2 | Pneumonie | 189 |
| XM-049849 | 0,0006 | 0,219 | -0.'174 | Hs.512898 | 2 | Pneumonie | 190 |
| XM-057131 | 0,0000 | -0,313 | 0,248 | Hs.525157 | 2 | Pneumonie | 191 |

**Tabelle 4: Gemeinsamer Set an Sequenzen mit ähnlicher signifikanter Genaktivität bei Patienten mit schweren Infektionen, die spezifisch für eine lokale Entzündung, nicht aber für Peritonitis oder Pneumonie, eines "Fiebers unklarer Genese"sind.**

| | | | **Normalisierte und transformierte Expressionssignale** | | | | | |
|---|---|---|---|---|---|---|---|---|
| **GenBank Accession Nummer** | **p value (Patientengruppe 1a)** | **p value (Patientengruppe 1b)** | **mean Patientengruppe 2** | **mean Patientengruppe 1a** | **mean Patientengruppe 1b** | **UniGene** | **Cluster** | **SeqID** |
| AA035428 | 0,006 | 0,007 | 0,15 | -0,17 | -0,15 | Hs.437006 | 3 | 192 |
| AA044390 | 0,015 | 0 | -0,22 | 0,27 | 0,27 | Hs.516217 | 3 | 193 |
| AA046302 | 0,032 | 0 | 0,15 | -0,17 | -0,35 | Hs.21611 | 3 | 194 |
| AA059314 | 0,037 | 0,011 | 0,15 | -0,18 | -0,22 | Hs.433445 | 3 | 195 |
| AA151104 | 0,001 | 0 | 0,24 | -0,29 | -0,29 | Hs.73454 | 3 | 196 |
| AA397913 | 0,006 | 0,04 | -0,12 | 0,14 | 0,11 | Hs.549040 | 3 | 197 |
| AA398331 | 0,018 | 0,007 | 0,16 | -0,19 | -0,2 | Hs.599407 | 3 | 198 |
| AA400790 | 0,011 | 0,006 | 0,22 | -0,26 | -0,24 | Hs.127999 | 3 | 199 |
| AA418841 | 0,039 | 0,03 | -0,16 | 0,19 | 0,13 | Hs.475319 | 3 | 200 |
| AA425808 | 0 | 0,005 | 0,22 | -0,27 | -0,14 | | 3 | 201 |
| AA426618 | 0,008 | 0,007 | 0,16 | -0,19 | -0,15 | Hs.585398 | 3 | 202 |
| AA455638 | 0,045 | 0,007 | -0,14 | 0,17 | 0,19 | Hs.444332 | 3 | 203 |
| AA461499 | 0,029 | 0,014 | 0,14 | -0,16 | -0,15 | Hs.99546 | 3 | 204 |
| AA478985 | 0,012 | 0,008 | 0,17 | -0,2 | -0,15 | Hs.279784 | 3 | 205 |
| AA514237 | 0 | 0,001 | -0,23 | 0,28 | 0,19 | Hs.147880 | 3 | 206 |
| AA620760 | 0,007 | 0,016 | 0,21 | -0,25 | -0,21 | Hs.633241 | 3 | 207 |
| AA682790 | 0,002 | 0,04 | 0,16 | -0,19 | -0,11 | | 3 | 208 |
| AA702492 | 0 | 0,002 | -0,29 | 0,35 | 0,17 | Hs.368563 | 3 | 209 |
| AA703200 | 0,01 | 0,001 | 0,17 | -0,2 | -0,18 | Hs.597767 | 3 | 210 |
| AA740907 | 0,004 | 0 | 0,15 | -0,18 | -0,27 | Hs.88297 | 3 | 211 |
| AA759092 | 0,016 | 0,025 | 0,11 | -0,14 | -0,15 | Hs.121439 | 3 | 212 |
| AA781411 | 0,005 | 0,021 | 0,16 | -0,2 | -0,12 | Hs.166015 | 3 | 213 |
| AA807376 | 0,009 | 0,015 | 0,14 | -0,17 | -0.17 | Hs.370414 | 3 | 214 |
| AA813145 | 0,018 | 0 | 0,16 | -0,19 | -0,38 | Hs.601872 | 3 | 215 |
| AA845015 | 0,041 | 0,02 | 0,12 | -0,15 | -0,13 | Hs.631866 | 3 | 216 |
| AA845475 | 0 | 0,022 | -0,2 | 0,24 | 0,1 | Hs.487393 | 3 | 217 |
| AA860398 | 0,014 | 0,018 | 0,12 | -0,14 | -0,15 | Hs.569748 | 3 | 218 |
| AA894523 | 0,013 | 0 | 0,13 | -0,15 | -0,18 | Hs.10734 | 3 | 219 |
| AA947111 | 0,004 | 0,006 | 0,13 | -0,15 | -0,11 | Hs.562136 | 3 | 220 |
| ADRB1 | 0 | 0 | -0,2 | 0,24 | 0,25 | Mm.46797 | 3 | 221 |
| AHSG | 0,022 | 0,02 | 0,22 | -0,26 | -0,18 | Hs.324746 | 3 | 222 |
| AI023558 | 0,001 | 0,001 | 0,15 | -0,18 | -0,17 | Hs.131417 | 3 | 223 |
| AI041544 | 0 | 0 | -0,22 | 0,26 | 0,16 | Hs.575480 | 3 | 224 |
| AI091867 | 0 | 0 | -0,22 | 0,26 | 0,24 | Hs.631761 | 3 | 225 |
| AI093704 | 0,009 | 0,012 | -0,18 | 0,21 | 0,19 | Hs.603146 | 3 | 226 |
| AI097494 | 0,002 | 0,005 | 0,17 | -0,21 | -0,16 | Hs.307984 | 3 | 227 |
| AI148246 | 0,024 | 0,004 | 0,14 | -0,17 | -0,18 | Hs.474251 | 3 | 228 |
| AI167874 | 0,006 | 0,002 | 0,13 | -0,15 | -0,16 | Hs.71023 | 3 | 229 |
| AI203091 | 0,016 | 0,027 | 0,13 | -0,15 | -0,12 | Hs.604090 | 3 | 230 |
| AI203697 | 0,001 | 0,016 | -0,15 | 0,18 | 0,09 | Hs.567342 | 3 | 231 |
| AI220662 | 0,049 | 0,019 | -0,12 | 0,15 | 0,1 | Hs.508720 | 3 | 232 |
| AI222359 | 0,005 | 0,003 | 0,23 | -0,28 | -0,29 | Hs.556230 | 3 | 233 |
| AI223092 | 0,004 | 0,015 | 0,16 | -0,19 | -0,18 | Hs.147880 | 3 | 234 |
| AI264626 | 0,001 | 0,005 | 0,16 | -0,19 | -0,12 | | 3 | 235 |
| AI267659 | 0,026 | 0.031 | 0,13 | -0,15 | -0,11 | | 3 | 236 |
| AI271764 | 0 | 0,003 | -0,31 | 0,37 | 0,28 | Hs.446357 | 3 | 237 |
| AI342905 | 0,032 | 0,04 | 0,15 | -0,18 | -0,17 | Hs.604613 | 3 | 238 |
| AI357099 | 0,005 | 0,025 | 0,28 | -0,34 | -0,26 | Hs.584910 | 3 | 239 |
| AI373295 | 0,012 | 0,034 | 0,15 | -0,18 | -0,13 | Hs.544825 | 3 | 240 |
| AI373525 | 0,018 | 0,001 | 0,11 | -0,13 | -0,14 | Hs.539391 | 3 | 241 |
| AI378275 | 0,006 | 0,018 | 0,18 | -0,21 | -0,15 | Hs.61271 | 3 | 242 |
| AI453476 | 0,02 | 0,031 | 0,15 | -0,18 | -0,14 | Hs.168677 | 3 | 243 |
| AI478776 | 0,001 | 0,042 | -0,18 | 0,22 | 0,15 | | 3 | 244 |
| AI539271 | 0,027 | 0,016 | 0,19 | -0,23 | -0,23 | Hs.478000 | 3 | 245 |
| AI554283 | 0,043 | 0,021 | 0,14 | -0,17 | -0,16 | Hs.420529 | 3 | 246 |
| AI565469 | 0,015 | 0 | 0,16 | -0,19 | -0,22 | Hs.638685 | 3 | 247 |
| AI582909 | 0 | 0 | 0,2 | -0,24 | -0,22 | Hs.578450 | 3 | 248 |
| AI589096 | 0,002 | 0,036 | -0,23 | 0,27 | 0,18 | Hs.638946 | 3 | 249 |
| AI590144 | 0,003 | 0,022 | 0,27 | -0,33 | -0,24 | Hs.508848 | 3 | 250 |
| AI613038 | 0,012 | 0 | -0,15 | 0,17 | 0,26 | | 3 | 251 |
| AI627286 | 0,008 | 0,003 | 0,12 | -0,14 | -0,14 | Hs.191073 | 3 | 252 |
| AI628322 | 0,006 | 0,038 | 0,22 | -0,27 | -0,12 | Hs.530538 | 3 | 253 |
| AI652609 | 0,027 | 0,012 | 0,12 | -0,14 | -0,12 | Hs.567425 | 3 | 254 |
| AI692869 | 0,002 | 0,028 | 0,24 | -0,28 | -0,19 | Hs.202419 | 3 | 255 |
| AI696291 | 0,027 | 0,033 | 0,21 | -0,25 | -0,25 | Hs,528671 | 3 | 256 |
| AI700444 | 0,029 | 0,019 | 0,18 | -0,21 | -0,23 | Hs.564343 | 3 | 257 |
| AI733177 | 0 | 0,004 | -0,22 | 0,27 | 0,22 | Hs.421340 | 3 | 258 |
| AI733269 | 0 | 0,003 | -0,26 | 0,31 | 0,18 | Hs.418045 | 3 | 259 |
| AI733498 | 0,045 | 0,001 | 0,16 | -0,19 | -0,32 | Hs.573606 | 3 | 260 |
| AI738831 | 0 | 0,032 | 0,26 | -0,32 | -0,14 | Hs.268606 | 3 | 261 |
| AI739381 | 0,005 | 0,004 | 0,14 | -0,16 | -0,2 | Hs.512763 | 3 | 262 |
| AI742529 | 0 | 0,003 | -0,22 | 0,27 | 0,16 | Hs.591095 | 3 | 263 |
| AI799137 | 0,048 | 0,006 | -0,16 | 0,19 | 0,27 | Hs.432690 | 3 | 264 |
| AI808410 | 0,006 | 0,008 | -0,16 | 0,19 | 0,15 | Hs.233955 | 3 | 265 |
| AI859370 | 0,027 | 0,015 | 0,12 | -0,14 | -0,14 | Hs.607181 | 3 | 266 |
| AI859777 | 0,013 | 0,011 | 0,22 | -0,26 | -0,25 | Hs.301819 | 3 | 267 |
| AI860121 | 0,001 | 0,004 | -0,21 | 0,25 | 0,23 | Hs.501684 | 3 | 268 |
| AI889310 | 0 | 0 | -0,21 | 0,25 | 0.17 | Hs.511903 | 3 | 269 |
| AI890962 | 0,026 | 0,017 | 0,13 | -0,16 | -0.12 | Hs.25601 | 3 | 270 |
| AI913322 | 0,009 | 0,023 | -0,24 | 0,29 | 0,19 | Hs.107740 | 3 | 271 |
| AI924028 | 0,003 | 0,003 | 0,28 | -0,34 | -0,27 | Hs.326391 | 3 | 272 |
| AI924296 | 0,017 | 0,041 | 0,11 | -0,13 | -0,11 | Hs.487479 | 3 | 273 |
| AI925451 | 0,046 | 0,008 | 0,16 | -0,19 | -0,18 | Hs.629605 | 3 | 274 |
| AI926659 | 0,001 | 0,04 | -0,23 | 0.27 | 0,15 | Hs.363558 | 3 | 275 |
| AI933607 | 0 | 0,002 | -0,25 | 0,31 | 0,17 | Hs.632296 | 3 | 276 |
| AI936462 | 0,016 | 0,012 | -0,13 | 0,16 | 0,11 | | 3 | 277 |
| ARHA | 0,001 | 0,004 | -0,18 | 0,22 | 0,13 | Hs.247077 | 3 | 278 |
| ASC | 0,001 | 0,014 | -0.2 | 0,24 | 0,11 | Hs.499094 | 3 | 279 |
| B3GALT3 | 0,005 | 0,01 | 0,21 | -0,25 | -0,18 | Hs.418062 | 3 | 280 |
| BC013302 | 0,003 | 0,033 | -0,13 | 0,16 | 0,08 | Hs.437594 | 3 | 281 |
| BC021289 | 0,023 | 0,005 | -0,27 | 0,33 | 0,27 | Hs.75431 | 3 | 282 |
| BC024270 | 0 | 0,017 | -0,15 | 0,18 | 0,13 | Hs.136164 | 3 | 283 |
| BCL6 | 0,003 | 0,011 | -0,26 | 0,32 | 0,21 | Hs.636990 | 3 | 284 |
| BZRP | 0 | 0 | -0,47 | 0,56 | 0,37 | Hs.202 | 3 | 285 |
| BZRP.1 | 0,002 | 0 | -0,34 | 0,41 | 0,35 | Hs.202 | 3 | 286 |
| C1QBP | 0 | 0,001 | -0,24 | 0,28 | 0,23 | Hs.622699 | 3 | 287 |
| C1S | 0,015 | 0,037 | 0,12 | -0,15 | -0,09 | Hs.458355 | 3 | 288 |
| C8B | 0,005 | 0 | 0,13 | -0,16 | -0,2 | Hs.391835 | 3 | 289 |
| C9 | 0 | 0,001 | -0,25 | 0,3 | 0,15 | Hs.481980 | 3 | 290 |
| CCR7 | 0,037 | 0,024 | 0,11 | -0,13 | -0,12 | Hs.370036 | 3 | 291 |
| CD59 | 0,007 | 0,015 | -0,2 | 0,24 | 0,17 | Hs.633297 | 3 | 292 |
| CDKN1A | 0,049 | 0,007 | -0,12 | 0,14 | 0,18 | Hs.370771 | 3 | 293 |
| CKM | 0,004 | 0,003 | 0,2 | -0,24 | -0,22 | Hs.334347 | 3 | 294 |
| EDARADD | 0 | 0 | -0,24 | 0,29 | 0,2 | Hs.352224 | 3 | 295 |
| EPB41 | 0,018 | 0,013 | 0,2 | -0,25 | -0,25 | Hs.175437 | 3 | 296 |
| FADD | 0 | 0,009 | -0,21 | 0,25 | 0,12 | Hs.86131 | 3 | 297 |
| FLOT2 | 0 | 0 | -0,35 | 0,42 | 0,29 | Hs.514038 | 3 | 298 |
| G3BP2 | 0,001 | 0.003 | -0,19 | 0,23 | 0,22 | Hs.593867 | 3 | 299 |
| GADD45A | 0,001 | 0,007 | -0,27 | 0,33 | 0,21 | Hs.80409 | 3 | 300 |
| H16716 | 0,002 | 0 | -0,25 | 0,31 | 0.25 | Hs.448889 | 3 | 301 |
| H18435 | 0,012 | 0,001 | 0,18 | -0,22 | -0,31 | | 3 | 302 |
| H18649 | 0 | 0,007 | 0,29 | -0,34 | -0,19 | | 3 | 303 |
| H23819 | 0,015 | 0,004 | 0,17 | -0,2 | -0,22 | Hs.59093 | 3 | 304 |
| H30516 | 0,012 | 0,031 | 0.17 | -0.2 | -0,14 | Hs.482411 | 3 | 305 |
| H44908 | 0 | 0 | -0,28 | 0,34 | 0,23 | Hs.591171 | 3 | 306 |
| H61449 | 0,009 | 0 | 0,13 | -0,15 | -0,18 | Hs.528368 | 3 | 307 |
| H98244 | 0,001 | 0,019 | 0,23 | -0,28 | -0,17 | Hs.143707 | 3 | 308 |
| H99099 | 0,003 | 0,029 | -0,22 | 0,26 | 0,17 | Hs.205742 | 3 | 309 |
| HSPA1A | 0,011 | 0,019 | -0,16 | 0,19 | 0,13 | Hs.520028 | 3 | 310 |
| HSPA8.1 | 0,005 | 0,006 | -0,2 | 0,23 | 0,23 | Hs.180414 | 3 | 311 |
| HSPB1 | 0 | 0,013 | -0,41 | 0,5 | 0,14 | Hs.520973 | 3 | 312 |
| ICAM5 | 0,003 | 0,003 | -0,13 | 0,15 | 0,13 | Hs.465862 | 3 | 313 |
| IFNA5 | 0,012 | 0 | -0,16 | 0,19 | 0,21 | Hs.37113 | 3 | 314 |
| IKBKG | 0,001 | 0 | -0,13 | 0,16 | 0,14 | Hs.43505 | 3 | 315 |
| IL10RB | 0 | 0,004 | -0,23 | 0,28 | 0,17 | Hs.512211 | 3 | 316 |
| IL12B | 0,036 | 0,015 | 0,32 | -0,39 | -0,28 | Hs.674 | 3 | 317 |
| IL1RN | 0,002 | 0 | -0,31 | 0,37 | 0,27 | Hs.594611 | 3 | 318 |
| IL2RG | 0 | 0,013 | -0,24 | 0,29 | 0,12 | Hs.84 | 3 | 319 |
| IL6R | 0,002 | 0,001 | -0,16 | 0,19 | 0,19 | Hs.591492 | 3 | 320 |
| IL7R | 0,019 | 0,04 | 0,21 | -0,26 | -0,17 | Hs.591742 | 3 | 321 |
| M37435 | 0,013 | 0,017 | -0,13 | 0,15 | 0,13 | Hs.173894 | 3 | 322 |
| M61199 | 0,003 | 0,028 | -0,22 | 0,26 | 0,16 | Hs.591602 | 3 | 323 |
| MAP3K11 | 0,024 | 0,01 | -0,14 | 0,16 | 0,13 | Hs.502872 | 3 | 324 |
| MAP3K6 | 0,032 | 0,041 | -0,12 | 0,15 | 0,11 | Hs.194694 | 3 | 325 |
| MAPK14 | 0 | 0,004 | -0,24 | 0,28 | 0,19 | Hs.588289 | 3 | 326 |
| MAPK14.3 | 0 | 0,001 | -0,26 | 0,31 | 0,25 | Hs.588289 | 3 | 327 |
| MAPK8.3 | 0,025 | 0,016 | 0,11 | -0,13 | -0,14 | Hs.138211 | 3 | 328 |
| MYD88 | 0 | 0 | -0,25 | 0,3 | 0,25 | Hs.82116 | 3 | 329 |
| MYL2 | 0,032 | 0 | -0,17 | 0,2 | 0,23 | Hs.491359 | 3 | 330 |
| N32857 | 0,004 | 0,02 | -0,54 | 0,65 | 0,45 | Hs.491767 | 3 | 331 |
| N51537 | 0,042 | 0,002 | 0,15 | -0,18 | -0,19 | Hs.525485 | 3 | 332 |
| N52915 | 0,005 | 0 | 0,16 | -0,19 | -0,18 | | 3 | 333 |
| N53973 | 0,014 | 0,006 | 0,13 | -0,16 | -0,15 | Hs.585782 | 3 | 334 |
| N67859 | 0,004 | 0,041 | -0,16 | 0,19 | 0,12 | Hs.125829 | 3 | 335 |
| NET1 | 0,021 | 0,013 | -0,11 | 0,13 | 0,13 | Hs.610771 | 3 | 336 |
| NM_001540 | 0 | 0,013 | -0,2 | 0,24 | 0,13 | Hs.626419 | 3 | 337 |
| NM_003258 | 0,005 | 0 | -0,21 | 0,25 | 0,27 | Hs.515122 | 3 | 338 |
| NM_006936 | 0,001 | 0,001 | -0,25 | 0,3 | 0,2 | Hs.474005 | 3 | 339 |
| NM_016184 | 0,017 | 0,022 | 0,22 | -0,27 | -0,24 | Hs.504657 | 3 | 340 |
| NM_025139 | 0,014 | 0,003 | 0,19 | -0,23 | -0,29 | Hs.471610 | 3 | 341 |
| NM_031311 | 0 | 0,024 | 0,5 | -0,6 | -0,24 | Hs.233389 | 3 | 342 |
| NM-000061 | 0,005 | 0,047 | -0,15 | 0,18 | 0,1 | Hs.159494 | 3 | 343 |
| NM-000584 | 0,017 | 0,007 | -0,18 | 0,21 | 0,21 | Hs.632880 | 3 | 344 |
| NM-000760 | 0,002 | 0,011 | -0,18 | 0,21 | 0,13 | Hs.524517 | 3 | 345 |
| NM-001013 | 0,015 | 0,027 | -0,11 | 0,13 | 0,11 | Hs.546288 | 3 | 346 |
| NM-001101 | 0 | 0 | -0,27 | 0,33 | 0,19 | Hs.593869 | 3 | 347 |
| NM-001288 | 0,007 | 0,001 | -0,14 | 0,17 | 0,18 | Mm.388801 | 3 | 348 |
| NM-001315 | 0 | 0,001 | -0,25 | 0,3 | 0,22 | Hs.588289 | 3 | 349 |
| NM-001569 | 0,001 | 0,012 | -0,16 | 0,19 | 0,11 | Hs.522819 | 3 | 350 |
| NM-001765 | 0,041 | 0,005 | -0,12 | 0,14 | 0,16 | Hs.1311 | 3 | 351 |
| NM-001772 | 0,018 | 0,004 | -0,12 | 0,14 | 0,12 | Hs.83731 | 3 | 352 |
| NM-002128 | 0,009 | 0,044 | -0,2 | 0,24 | 0,15 | Hs.593339 | 3 | 353 |
| NM-002394 | 0 | 0,012 | -0.19 | 0,23 | 0,12 | Hs.502769 | 3 | 354 |
| NM-002401 | 0,037 | 0.003 | -0,14 | 0,17 | 0,17 | Hs.29282 | 3 | 355 |
| NM-002415 | 0 | 0,025 | -0,24 | 0,28 | 0,14 | Hs.632781 | 3 | 356 |
| NM-002953 | 0 | 0 | -0,19 | 0,23 | 0,21 | Hs.149957 | 3 | 357 |
| NM-003153 | 0 | 0,041 | -0,2 | 0,24 | 0,13 | Hs.524518 | 3 | 358 |
| NM-003268 | 0 | 0,005 | -0,31 | 0,38 | 0,45 | Hs.135853 | 3 | 359 |
| NM-003684 | 0,017 | 0,001 | -0,17 | 0,2 | 0,16 | Hs.371594 | 3 | 360 |
| NM-004257 | 0 | 0 | -0,25 | 0,3 | 0,19 | Hs.446350 | 3 | 361 |
| NM-004635 | 0,02 | 0,031 | -0.1 | 0,12 | 0,11 | Hs.624942 | 3 | 362 |
| NM-004740 | 0 | 0,001 | -0,25 | 0,3 | 0,19 | Hs.462590 | 3 | 363 |
| NM-005620 | 0,001 | 0,001 | -0,23 | 0,27 | 0,22 | Hs.547382 | 3 | 364 |
| NM-005803 | 0,002 | 0,001 | -0,15 | 0,18 | 0,15 | Hs.179986 | 3 | 365 |
| NM-007328 | 0,005 | 0 | 0,36 | -0,43 | -0,38 | Hs.512576 | 3 | 366 |
| NM-018643 | 0 | 0,035 | 0,39 | -0,47 | -0,14 | Hs.283022 | 3 | 367 |
| NM-138556 | 0,03 | 0,009 | 0,17 | -0,2 | -0,1 | Hs.174312 | 3 | 368 |
| PPGB | 0 | 0,015 | -0,22 | 0,26 | 0,14 | Hs.609336 | 3 | 369 |
| PPP4C | 0 | 0 | -0,19 | 0,23 | 0,15 | Hs.534338 | 3 | 370 |
| PRP4.1 | 0,035 | 0,005 | -0,2 | 0,24 | 0,24 | Mm.10027 | 3 | 371 |
| R00742 | 0,007 | 0,037 | 0,16 | -0,19 | -0,15 | Hs.275675 | 3 | 372 |
| R05804 | 0,034 | 0,02 | 0,12 | -0,15 | -0,14 | Hs.348308 | 3 | 373 |
| R26635 | 0 | 0,041 | -0,49 | 0,59 | 0,16 | Hs.178499 | 3 | 374 |
| R37251 | 0,033 | 0,038 | -0,16 | 0,19 | 0,17 | Hs.479099 | 3 | 375 |
| R39782 | 0 | 0,002 | 0,22 | -0,26 | -0,2 | Hs.21145 | 3 | 376 |
| R40406 | 0,022 | 0,035 | -0,14 | 0,16 | 0,2 | Hs.2853 | 3 | 377 |
| R42461 | 0,004 | 0,008 | -0,2 | 0,23 | 0,26 | Hs.579115 | 3 | 378 |
| R42782 | 0,026 | 0,017 | -0,17 | 0,21 | 0,14 | Hs.434971 | 3 | 379 |
| R43203 | 0,011 | 0,027 | 0,3 | -0,36 | -0,25 | Hs.73828 | 3 | 380 |
| R43722 | 0,033 | 0,032 | -0,2 | 0,23 | 0,21 | | 3 | 381 |
| R45159 | 0 | 0 | 0,23 | -0,28 | -0,22 | Hs.271285 | 3 | 382 |
| R45355 | 0 | 0 | -0,18 | 0,21 | 0,2 | Hs.201805 | 3 | 383 |
| R52949 | 0,024 | 0,035 | 0,16 | -0,19 | -0,17 | Hs.622398 | 3 | 384 |
| R54442 | 0,001 | 0,006 | 0,24 | -0,28 | -0,23 | Hs.416139 | 3 | 385 |
| R56890 | 0,011 | 0,002 | 0,14 | -0,16 | -0,14 | Hs.592205 | 3 | 386 |
| R58974 | 0,033 | 0,034 | 0,13 | -0,16 | -0,2 | Hs.335205 | 3 | 387 |
| R84393 | 0,027 | 0,023 | 0,15 | -0,17 | -0,11 | Hs.387255 | 3 | 388 |
| R89802 | 0,006 | 0 | 0,18 | -0,22 | -0,17 | Hs.93670 | 3 | 389 |
| RAC1 | 0,01 | 0,01 | -0,11 | 0,13 | 0,14 | Hs.413812 | 3 | 390 |
| RAC3 | 0 | 0 | -0,35 | 0,41 | 0,26 | Hs.45002 | 3 | 391 |
| SERCA2-2 | 0 | 0,004 | -0,17 | 0,2 | 0,15 | Hs.633656 | 3 | 392 |
| SPTLC2 | 0 | 0,007 | -0,19 | 0,23 | 0,2 | Hs.435661 | 3 | 393 |
| T65410 | 0,018 | 0,042 | -0,2 | 0,24 | 0,19 | Hs.125116 | 3 | 394 |
| T90460 | 0,005 | 0,023 | -0,15 | 0,18 | 0,12 | Hs.102471 | 3 | 395 |
| T91086 | 0,044 | 0,015 | 0,2 | -0,24 | -0,21 | | 3 | 396 |
| T91795 | 0,038 | 0,041 | 0,14 | -0,17 | -0,16 | Hs.502872 | 3 | 397 |
| T95815 | 0,038 | 0,032 | 0,09 | -0,11 | -0,12 | Hs.336994 | 3 | 398 |
| TNFSF14 | 0,004 | 0,001 | 0,13 | -0,16 | -0,17 | Hs.129708 | 3 | 399 |
| TNFSF9 | 0,001 | 0,03 | -0,19 | 0,22 | 0,14 | Hs.1524 | 3 | 400 |
| TRAF3 | 0,014 | 0,041 | -0,15 | 0,18 | 0,11 | Hs.510528 | 3 | 401 |
| U07802 | 0,004 | 0,023 | 0,24 | -0,28 | -0,26 | Hs.503093 | 3 | 402 |
| UGCG | 0,009 | 0 | -0,26 | 0,31 | 0,53 | Hs.304249 | 3 | 403 |
| W86767 | 0,046 | 0,001 | 0,1 | -0,12 | -0,17 | Hs.632594 | 3 | 404 |
| X05875 | 0,011 | 0,021 | -0,37 | 0,45 | 0,23 | Hs.99863 | 3 | 405 |
| X64641 | 0,004 | 0,015 | -0,46 | 0,56 | 0,44 | Hs.449621 | 3 | 406 |
| XM-003937 | 0,002 | 0,001 | 0,26 | -0,32 | -0,38 | Hs.591258 | 3 | 407 |
| XM-004256 | 0,014 | 0 | -0,31 | 0,38 | 0,6 | Hs.606320 | 3 | 408 |
| XM-006867 | 0,002 | 0,001 | -0,19 | 0,22 | 0,19 | Hs.591014 | 3 | 409 |
| XM-007417 | 0,031 | 0,016 | 0,1 | -0,12 | -0,27 | Hs.636674 | 3 | 410 |
| XM-008679 | 0,009 | 0,019 | -0,28 | 0,33 | 0,24 | Hs.408312 | 3 | 411 |
| XM-009475 | 0,001 | 0,001 | -0,36 | 0,44 | 0,4 | Hs.388004 | 3 | 412 |
| XM-012039 | 0 | 0,004 | -0,28 | 0,33 | 0,24 | Hs.568921 | 3 | 413 |
| XM-015278 | 0 | 0,001 | -0,19 | 0,23 | 0,21 | Hs.334019 | 3 | 414 |
| XM-015396 | 0 | 0 | -0,41 | 0,49 | 0,35 | Hs.507658 | 3 | 415 |
| XM-015815 | 0,008 | 0,007 | -0,23 | 0,27 | 0,25 | Hs.509067 | 3 | 416 |
| XM-027358 | 0 | 0,042 | -0,29 | 0,35 | 0,16 | Hs. 592992 | 3 | 417 |
| XM-030326 | 0,002 | 0,021 | -0,22 | 0,26 | 0,17 | Hs.626357 | 3 | 418 |
| XM-030906 | 0 | 0 | -0,23 | 0,27 | 0,22 | Hs.155218 | 3 | 419 |
| XM-031242 | 0 | 0 | -0,25 | 0,3 | 0,29 | Hs.527778 | 3 | 420 |
| XM-033862 | 0,004 | 0,001 | -0,13 | 0,15 | 0,14 | Hs.181128 | 3 | 421 |
| XM-038024 | 0,024 | 0,033 | 0,14 | -0,17 | -0,11 | Hs.318547 | 3 | 422 |
| XM-041101 | 0,004 | 0 | -0,19 | 0,22 | 0,18 | Hs.504877 | 3 | 423 |
| XM-042066 | 0,006 | 0,025 | -0,14 | 0,17 | 0,15 | Hs.508461 | 3 | 424 |
| XM-046575 | 0,028 | 0,007 | 0,18 | -0,21 | -0,2 | Hs.562457 | 3 | 425 |
| XM-047570 | 0 | 0 | -0,26 | 0,32 | 0,21 | Hs.495912 | 3 | 426 |
| XM-048068 | 0,001 | 0 | -0,24 | 0,29 | 0,23 | Hs.531668 | 3 | 427 |
| XM-052636 | 0 | 0 | -0,27 | 0,32 | 0,23 | Hs.634911 | 3 | 428 |
| XM-055188 | 0,029 | 0,011 | -0,17 | 0,2 | 0,18 | Hs.76753 | 3 | 429 |
| XM-056556 | 0 | 0,019 | -0,34 | 0,41 | 0,17 | Hs.501497 | 3 | 430 |
| XM-057356 | 0 | 0,009 | -0,22 | 0,26 | 0,23 | Hs.268675 | 3 | 431 |
| XM-114018 | 0,001 | 0,012 | -0,17 | 0,21 | 0,12 | Hs.591382 | 3 | 432 |

Damit sind die ermittelten spezifischen Genaktivitätscluster 1 und 2 für die Feststellung von Peritonitis oder Pneumonie bei Sepsispatienten für die Erfindung anwendbar.

### Referenzen

1. Roth A.R., Basello, D.O., (2003), Approach to the adult patient with fever of unknown origin, Am. Fam. Phys.,68(11), 2223-2228.
2. Amin K., Kauffman C.A., (2003), Fever of unknown origin, postgrad. med., 114(3), 69-75.
3. Mourad, O., Palda, V., Detsky, A.S., (2003), A comprehensive evidence-based approach to fever of unknown origin, Arch. Intern. Med., 163, 545-551.
4. Liu, K.S., Shen, W.S., Chen, Y.C:, Chang, S.C., Hsieh, W.C., (2003), Fever of unknown origin: a retrospective study of 78 adult patients in Taiwan, J. Microbiol. Immunol. Infect., 36, 243-247.
5. Pile, J.C., (2006), Evaluating postoperative fever,: a focused approach, Clev. Clin. J. Med., 73 (supp.1), S62-S66.
6. Sauer, H-J., (2001), Surveillance nosokomialer Infektionen auf Intensivstationen-Etablierung einer computergestüzten Infektionserfassung und -auswertung auf einer interdiziplinären 16-Betten-Intensivstation, MD thesis, Halle (Saale), Martin-Luther-Universität Halle-Wittenberg, Medizinische Fakultät.
7. Vincent, J-L., BiharinD.J., Suter, P.M., Bruining, H.A.,White J., Nicolas-Chanoin, M-H., Wolff, M., Spencer, R.C:, Hemmer,M., (2000), teh prevalence of nosocomial infection in intensive care units in Europe: Results of the European preavalence of infection in intensive care (EPIC) study, JAMA, 37, 454-460.
8. Welte, T., Marre, R., Suttorp, N., (2004), Das Kompetenznetzwerk "Ambulant erworbene Pneumonie" (CAPNETZ), Internist, 45, 393-401.
9. Unertl, K. Heiniger, A., Ventilator-associated Pneumonia, http://www.tudresden.de/medkai/unertl.pdf
10. Patel, P.J., Leeper Jr, K.V., McGowan Jr, J.E., (2002). Epidemiology and microbiology of hospital-acquired pneumonia, Semin. Respir. Crit. Care Med.,23(5), 415-425.
11. Park, D.R., The microbiology of ventilator-associated Pneumonia, Respir. Care, 50(86), 742-765.
12. Hall, J.C., Heel, K.A., Papadimitriou, J.M., Platell C., (1998), The pathology of peritonitis, Gastroenterology, 114, 185-196.
13.Troidle, L., Gorban-Brennan, N., Kliger, A., Finkelstein, F., (1998), Differing outcomes of Gram-positive and Gram-negative peritonitis, Am. J. Kidney Dis., 32(4), 623-628.
14.Jönsson, B., Berlund, J., Skau, T., Nyström, P. O., (1993), Outcome of intrabdominal infection in pigs depends more on host repsonses than on microbiology, Eur. J. Surg., 159, 571-578.
15. Cobb, P.J., Laramie, J.,M., Stormo, G.D., Morrissey, J.J., Shannon, W.D., Qiu, Y., Karl, I., Buchman, T.G., Hotchkiss, R.S., (2002), Sepsis gene expression profiling: Murine splenic compared with hepatic responses determined using complementary microarrays, Crit Care Med.,30(12), 2711-2721.
16. Jonhson, S.B., (2006), Gene expression profiles differentiate between SIRS and early Sepsis, 126th Annual Meeting, American Surgical Assoc., Boston.
17. Mclean, A., (2006), Use of signature genes to diagnose sepsis in patients with SIRS, 26th International Symposium on Intensive Care and Emergency Medicine, Brussels.
18. Prucha M, Ruryk A, Boriss H, Moller E, Zazula R, Herold I, Claus RA, Reinhart KA, Deigner P, Russwurm S., (2004), Expression profiling: toward an application in sepsis diagnostics, Shock, 22(1):29-33.
19. Domashowske J.B., Bonville C.A., Easton, A.J., Rosenberg H.F., (2002), Differential expression of pro-inflammatory cytokine genes in vivo in response to pathogenic and non-pathogenic pneumovirus infections., J. Infect. Dis., 186(1), 8-14.
20. WO 2004/087949 , Verfahren zur Erkennung akuter, generalisierter entzündlicher Zustände (SIRS), Sepsis, sepsisähnlichen Zuständen und systemischen Infektionen
21. WO 03/002763, Use of a biochip for the diagnosis of sepsis or sepsis related syndrome
22. DE 102004049897, Verfahren zur Unterscheidung zwischen nichtinfektiösen und infektiösen Ursachen eines Multiorganversagens
23. Marshall, D.R., Olivas, E., Andreansky, S., La Gruta, N.L., Neale, G.A., Gutierrez, A., Wichlan, D.G., Cheng, C., Doherty, P.C.,Turner, S.J., (2005), Effector CD8+ T cells recovered from influenza pneumonia differentiate to state of focused gene expression, Proc. Natl. Acad. Sci. USA, 102(17), 6074-6079.
24.Selman, M., pardo, A., Barrera, L., Estradda, A., Watson, S.R., Wilson,K., Aziz, N., Kaminski, N., Zlotnik A., (2006), Gene expression profiles distinguish idiopathic pulmonary fibrosis from hypersensistivity pneumonitis, Am J. respir. Crit. Care Med., 173(2), 188-198.
25. Sandler, N.G., Menttink-Kane, M.M., Cheever, A.W., Wynn, T.A., (2003), Global gene expression profiles during acute pathogen-induced pulmonary inflammation reveal divergent roles for Th1 snf Th2 responses in tissue repair, J. Immunol.,171, 3655-3667.
26. Abdrejko, K.M, Deutschman, C.S, (1997), Altered hepatic gene expression in fecal peritonitis: changes in transcription of glucogenic, beta-oxidative, and ureagenic genes, Schock, 7(3), 164-169.
27. US2006/0040301, Diagnostic assay for source of inflammation
28. Rheinart, K., (2005), The genetic response to sepsis - Can we use it to improve diagnosis?, 25th International Symposium on Intensive Care and Emergency Medicine, Brussels.
29. DE 102005013013, Verwendung von Geneaktivitäts-Klassifikatoren für die in vitro Klassifizierung von Genexpressionsprofilen von Patienten mit infektiösem/nichtinfektiösem Multiorganversagen
30. Bone RC, Balk RA, Cerra FB, et al. (1992) The ACCP/SCCM Consensus Conference Committee (1992) Definitions for Sepsis and organ failure and guidelines for the use of innovative therapies in Sepsis. Chest 101:1656-1662; und Crit Care Med 1992; 20: 864-874.
31. Huber W, Heydebreck A, Sueltmann H, et al. (2003) Parameter estimation for the calibration and variance stabilization of microarray data. Stat. Appl. in Gen. and Mol. Biol.. Vol. 2, Issue 1, Article 3

## Patentansprüche

1. Verwendung von in vitro aus einer Patientenblutprobe erhaltenen Genexpressionsprofilen für die Feststellung von Peritonitis und Pneumonie bei Sepsispatienten, wobei die zur Erstellung verwendeten Polynukleotide, die in ihrem Expressionsverhalten vergleichbare Genaktivitäten aufweisen, zu diagnostischen Genaktivitätsclustern zusammengefasst werden und wobei die diagnostischen Genaktivitätscluster sich wie folgt zusammensetzen:
Cluster 1: SEQ-ID No.1 bis SEQ-ID No. 77
Cluster 2: SEQ-ID No. 78 bis SEQ-ID No. 191
wobei die Polynukleotide der SEQ-IDs 1 bis 77 spezifisch für Peritonitis sind, die Polynukleotide der SEQ-IDs 78 bis SEQ-ID No. 191 spezifisch für Pneumonie sind,
wobei SEQ-IDs 1-4, 7-9, 11, 14-16, 21, 23, 26-29, 32, 34-38, 41-49, 51, 52, 54-63, 65, 67, 69-71, 73-75, 77 bei Peritonitis überexprimiert Werden, und SEQ-IDs 5, 6, 10, 12, 13, 17-20, 22, 24, 25, 30, 31, 33, 39, 40, 50, 53, 64, 66, 68, 72, 76. bei Peritonitis unterexprimiert werden, und
wobei SEQ-IDs 80, 85-87, 91, 94, 97, 98, 100, 107, 111, 112, 117, 118, 120, 121, 123, 124, 127-129, 131, 133-135, 138, 142, 145, 149, 151-154, 156, 159, 162, 164, 165, 175, 182, 184, 186, 187, 191 bei Pneumonie überexprimiert werden, und SEQ-IDs 78, 79, 81-84, 88-90, 92, 93, 95, 96, 99, 101-106, 108-110, 113-116, 119, 122, 125, 126, 130, 132, 136, 137, 139-141, 143, 144, 146-148, 150, 155, 157, 158, 160, 161, 163, 167-174, 176-181, 183, 185, 188-190 bei Pneumonie unterexprimiert werden.

2. Verwendung nach Anspruch 1 als Ein- oder Ausschlusskriterium in klinischen Studien und/oder zur Erstellung von Genaktivitätsdaten für die elektronische Weiterverarbeitung.

3. Verwendung nach einem der Ansprüche 1 bis 2, wobei die erhaltenen Genaktivitätsdaten zur Herstellung von Software für die Beschreibung der individuellen Prognose eines Patienten, für Diagnosezwecke und/oder Patientendatenmangementsysteme eingesetzt werden, und/oder die in-vitro aus einer Patientenblutprobe erhaltenen Genexpressionsprofile zur Herstellung von klinischen Expertensystemen und/oder zur Modellierung von zellulären Signalübertragungswegen eingesetzt werden.

4. Verwendung nach einem der Ansprüche 1 bis 3, wobei zur Erstellung des Genexpressionsprofiles Gen und/oder Genfragment verwendet wird, welches ausgewählt wird aus der Gruppe bestehend aus SEQ-ID No. 1 bis SEQ-ID No. 191, Genfragmenten davon mit wenigstens 20 Nukleotiden sowie Genen mit einer Sequenzhomologie von mindestens 80%.

5. Verwendung nach einem der Ansprüche 1 bis 4, **dadurch gekennzeichnet, dass** die Genexpressionsprofile mittels Hybridisierungsverfahren, insbesondere solchen auf Microarrays oder real-time PCR basiert, ermittelt werden.

6. Verfahren zur in vitro Messung von Genexpressionsprofilen und/oder mindestens einem Genaktivitätscluster zur Feststellung von Peritonitis und Pneumonie bei Sepsispatienten, **dadurch gekennzeichnet, dass** man in Patientenblutprobe die Genaktivität einer Mehrzahl von bestimmten, mit der Peritonitis und Pneumonie bei Sepsis in Zusammenhang stehenden Genen in einer Patientenblutprobe bestimmt, wobei die Gene ausgewählt werden aus der Gruppe bestehend aus: SEQ-ID No.1 bis SEQ-ID No. 191, Genfragmenten davon mit wenigstens 20 Nukleotiden sowie Genen mit einer Sequenzhomologie von mindestens 80% und zu folgenden diagnostischen Clustern zusammengefasst werden:
Cluster 1: SEQ-ID No.1 bis SEQ-ID No. 77
Cluster 2: SEQ-ID No. 78 bis SEQ-ID No. 191.
wobei die Polynukleotide der SEQ-IDs 1 bis 77 spezifisch für Peritonitis sind, die Polynukleotide der SEQ-IDs 78 bis SEQ-ID No. 191 spezifisch für Pneumonie sind,
wobei SEQ-IDs 1-4, 7-9, 11, 14-16, 21, 23, 26-29, 32, 34-38, 41-49, 51, 52, 54-63, 65, 67, 69-71, 73-75, 77 bei Peritonitis überexprimiert werden, und SEQ-IDs 5, 6, 10, 12, 13, 17-20, 22, 24, 25, 30, 31, 33, 39, 40, 50, 53, 64, 66, 68, 72, 76. bei Peritonitis unterexprimiert werden, und
wobei SEQ-IDs 80, 85-87, 91, 94, 97, 98, 100, 107, 111, 112, 117, 118, 120, 121, 123, 124, 127-129, 131, 133-135, 138, 142, 145, 149, 151-154, 156, 159, 162, 164, 165, 175, 182, 184, 186, 187, 191 bei Pneumonie überexprimiert werden, und SEQ-IDs 78, 79, 81-84, 88-90, 92, 93, 95, 96, 99, 101-106, 108-110, 113-116, 119, 122, 125, 126, 130, 132, 136, 137, 139-141, 143, 144, 146-148, 150, 155, 157, 158, 160, 161, 163, 167-174, 176-181, 183, 185, 188-190 bei Pneumonie unterexprimiert werden.

7. Verfahren nach Anspruch 7, **dadurch gekennzeichnet, dass** die in Anspruch 6 aufgelisteten Gene oder Genfragmente und/oder von deren RNA abgeleiteten Sequenzen ersetzt werden durch: synthetische Analoga, Aptamere, Spiegelmere sowie Peptido- und Morpholinonukleinsäuren.

8. Verfahren nach Anspruch 6 oder 7, **dadurch gekennzeichnet, dass** die Genaktivitäten mittels Hybridisierungsverfahren und/oder mittels Microarrays bestimmt werden, und/oder durch Hybridisierungs-unabhängige Verfahren, insbesondere enzymatische und/oder chemische Hydrolyse und/oder Amplifikationsverfahren, vorzugsweise PCR, anschließende Quantifizierung der Nukleinsäuren und/oder von Derivaten und/oder Fragmenten derselben, bestimmt werden.

9. Verwendung von in vitro aus einer Patientenblutprobe erhaltenen Genexpressionsprofilen und/oder von den hierfür verwendeten Sonden, welche ausgewählt werden aus der Gruppe bestehend aus SEQ-ID No. 1 bis SEQ-ID No. 191 sowie Genfragmenten davon mit wenigstens 20 Nukleotiden, zur Bestimmung der Genaktivität oder den davon abgeleiteten Proteinprodukten zum Screening von Wirkstoffen gegen Peritonitis und/oder Pneumonie und/oder zur Beurteilung der Therapieeffekte von Wirkstoffen gegen Peritonitis und/oder Pneumonie.

10. Verwendung nach Anspruch 9, **dadurch gekennzeichnet, dass** hybridisierungsfähige synthetische Analoga der in Anspruch 8 aufgelisteten Sonden verwendet werden.

11. Kit, enthaltend eine Auswahl von Sequenzen gemäß SEQ-ID No. 1 bis SEQ-ID No. 191, die spezifisch für die Feststellung von Peritonitis und Pneumonie bei Sepsis sind, und/oder Genfragmenten davon mit wenigstens 20 Nukleotiden zur Bestimmung von Genexpressionsprofilen in vitro in einer Patientenblutprobe, für die Feststellung Peritonitis und Pneumonie bei Sepsispatienten.

## Claims

1. Use of gene expression profiles obtained in vitro from a patient's blood sample for establishing peritonitis and pneumonia in sepsis patients, wherein polynucleotides used for establishing said gene expression profiles which show similar gene activity data in their expression behavior are grouped in diagnostic gene activity clusters and wherein the diagnostic gene activity clusters are composed as follows:
Cluster 1: SEQ-ID NO.1 to SEQ-ID No. 77
Cluster 2: SEQ-ID No. 78 to SEQ-ID No. 191
wherein the polynucleotides of SEQ-IDs 1 to 77 are specific for peritonitis, the polynucleotides of SEQ-IDs 78 to SEQ-ID No. 191 are specific for pneumonia,
wherein SEQ-IDs 1-4, 7-9, 11, 14-16, 21, 23, 26-29, 32, 34-38, 41-49, 51, 52, 54-63, 65, 67, 69-71, 73-75, 77 are over-expressed in case of peritonitis, and SEQ-I Ds 5, 6, 10, 12, 13, 17-20, 22, 24, 25, 30, 31, 33, 39, 40, 50, 53, 64, 66, 68, 72, 76 are under-expressed in case of peritonitis,
and wherein SEQ-IDs 80, 85-87, 91, 94, 97, 98, 100, 107, 111, 112, 117, 118, 120, 121, 123, 124, 127-129, 131, 133-135, 138, 142, 145, 149, 151-154, 156, 159, 162, 164, 165, 175, 182, 184, 186, 187, 191 are over-expressed in case of pneumonia, and SEQ-IDs 78, 79, 81-84, 88-90, 92, 93, 95, 96, 99, 101-106, 108-110, 113-116, 119, 122, 125, 126, 130, 132, 136, 137, 139-141, 143, 144, 146-148, 150, 155, 157, 158, 160, 161, 163, 167-174, 176-181, 183, 185, 188-190 are under-expressed in case of pneumonia.

2. The use according to claim 1 as inclusion or exclusion criterion in clinical studies and/or to establish gene activity data for electronic further processing.

3. The use according to one of the claims 1 to 2, wherein the gene activity data obtained are used for the production of software for the description of the individual prognosis of a patient, for diagnostic purposes and/or patient data management systems, and/or the gene expression profiles obtained in vitro from a patient's blood sample are used for the creation of clinical expert systems and/or for modeling cellular signal transduction pathways.

4. The use according to one of the claims 1 to 3, wherein a gene or gene fragment is used for generation of the gene expression profile, the gene or gene fragment being selected from the group consisting of SEQ-ID No. 1 to SEQ-ID No. 191, gene fragments thereof with at least 20 nucleotides and genes with a homology of sequence of at least 80%.

5. The use according to one of the claims 1 to 4, **characterized in that** the gene expression profiles are ascertained by means of hybridizing methods, in particular hybridizing methods based on microarrays or real-time PCR.

6. A method for in vitro measurement of gene expression profiles and/or at least one gene activity cluster for establishing peritonitis and pneumonia in sepsis patients, **characterized in that**, in a patient's blood sample, the gene activity of a plurality of certain genes related to peritonitis and pneumonia in case of sepsis is determined in a patient's blood sample, the genes being selected from a group consisting of: SEQ-ID No.1 to SEQ-ID No. 191, gene fragments thereof with at least 20 nucleotides as well as genes with a horology of sequence of at least 80%, and are grouped in diagnostic clusters as follows:
Cluster 1: SEQ-ID No.1 to SEQ-ID No. 77
Cluster 2: SEQ-ID No. 78 to SEQ-ID No. 191.
wherein the polynucleotides of SEQ-IDs 1 to 77 are specific for peritonitis, the polynucleotides of SEQ-IDs 78 to SEQ-ID No. 191 are specific for pneumonia,
wherein SEQ-IDs 1-4, 7-9, 11, 14-16, 21, 23, 26-29, 32, 34-38, 41-49, 51, 52, 54-63, 65, 67, 69-71, 73-75, 77 are over-expressed in case of peritonitis, and SEQ-IDs 5, 6, 10, 12, 13, 17-20, 22, 24, 25, 30, 31, 33, 39, 40, 50, 53, 64, 66, 68, 72, 76 are under-expressed in case of peritonitis,
and wherein SEQ-IDs 80, 85-87, 91, 94, 97, 98, 100, 107, 111, 112, 117, 118, 120, 121, 123, 124, 127-129, 131, 133-135, 138, 142, 145, 149, 151-154, 156, 159, 162, 164, 165, 175, 182, 184, 186, 187, 191 are over-expressed in case of pneumonia, and SEQ-IDs 78, 79, 81-84, 88-90, 92, 93, 95, 96, 99, 101-106, 108-110, 113-116, 119, 122, 125, 126, 130, 132, 136, 137, 139-141, 143, 144, 146-148, 150, 155, 157, 158, 160, 161, 163, 167-174, 176-181, 183, 185, 188-190 are under-expressed in case of pneumonia.

7. The method according to claim 6, **characterized in that** the genes or gene fragments stated in claim 6 and/or sequences derived from their RNA are replaced with a member selected from the group consisting of synthetic analogues, aptamers, Spiegelmers, peptido- and morpholinonucleic acids.

8. The method according to one of the claims 6 or 7, **characterized in that** the gene activity is determined by hybridization methods and/or microarrays, and/or hybridization-independent methods, in particular by enzymatic and/or chemical hydrolysis and/or amplification methods, preferably PCR, subsequent quantification of nucleic acids and/or derivates and/or fragments of the same.

9. Use of gene expression profiles that are obtained in vitro from a patient's blood sample and/or of probes used therefore, selected from the group consisting of SEQ-ID No. 1 to SEQ-ID No. 191 as well as gene fragments thereof with at least 20 nucleotides, for determining gene activity or of protein products derived therefrom for screening active agents against peritonitis and/or pneumonia, and/or wherein the gene expression profiles are used for evaluation of therapeutic effects of the active agents against peritonitis and/or pneumonia.

10. The use according to claim 9, **characterized in that** hybridizable synthetic analogues of the probes listed in claim 8 are used.

11. A kit containing a selection of sequences according to SEQ-ID No. 1 to SEQ-ID No. 191, which are specific for establishment of peritonitis and pneumonia in case of sepsis, and/or gene fragments thereof with at least 20 nucleotides for determination of gene expression profiles in vitro in a patient's blood sample, for use in determination of peritonitis and pneumonia in sepsis patients.

## Revendications

1. Utilisation de profils d'expression génique obtenus à partir d'un échantillon de sang d'un patient, pour la détection d'une péritonite et d'une pneumonie chez des patients septiques, les polynucléotides utilisés pour leur construction, qui présentent des activités géniques comparables pour ce qui est de leur comportement d'expression, étant regroupés en batteries d'activités géniques diagnostiques, et les batteries d'activités géniques diagnostiques ayant les compositions suivantes :
Batterie 1 : SEQ ID NO:1 à SEQ ID NO:77
Batterie 2 : SEQ ID NO:78 à SEQ ID NO:191
les polynucléotides SEQ ID 1 à 77 étant spécifiques de la péritonite, les polynucléotides SEQ ID 78 à SEQ ID NO:191 étant spécifiques de la pneumonie,
les séquences SEQ ID 1-4, 7-9, 11, 14-16, 21, 23, 26-29, 32, 34-38, 41-49, 51, 52, 54-63, 65, 67, 69-71, 73-75, 77 étant surexprimées dans le cas d'une péritonite, et les séquences SEQ ID 5, 6, 10, 12, 13, 17-20, 22, 24, 25, 30, 31, 33, 39, 40, 50, 53, 64, 66, 68, 72, 76 étant sous-exprimés dans le cas d'une péritonite, et
les séquences SEQ ID 80, 85-87, 91, 94, 97, 98, 100, 107, 111, 112, 117, 118, 120, 121, 123, 124, 127-129, 131, 133-135, 138, 142, 145, 149, 151-154, 156, 169, 162, 164, 165, 175, 182, 184, 186, 187, 191 étant surexprimées dans le cas d'une pneumonie, et les séquence SEQ ID 78, 79, 81-84, 88-90, 92, 93, 95, 96, 99, 101-106, 108-110, 113-116, 119, 122, 125, 126, 130, 132, 136, 137, 139-141, 143, 144, 146-148, 150, 155, 157, 158, 160, 161, 163, 167-174, 176-181, 183, 185, 188-190 étant sous-exprimées dans le cas d'une pneumonie.

2. Utilisation selon la revendication 1 en tant que critère d'inclusion ou d'exclusion dans des études cliniques et/ou pour fabriquer des données d'activités géniques pour le post-traitement électronique.

3. Utilisation selon l'une des revendications 1 à 2, pour laquelle les données d'activités géniques obtenues sont utilisées pour la préparation d'un logiciel pour la description du pronostic individuel d'un patient, à des fins de diagnostic et/ou de systèmes de gestion des données patients, et/ou les profils d'expression génique obtenus à partir d'un échantillon de sang d'un patient étant utilisés pour fabriquer des systèmes experts cliniques et/ou pour modéliser des voies cellulaires de transmission du signal.

4. Utilisation selon l'une des revendications 1 à 3, pour laquelle on utilise pour la construction du profil d'expression génique un gène et/ou un fragment génique qui est choisi dans le groupe consistant en SEQ ID NO:1 à SEQ ID NO:191, les fragments géniques de ces dernières ayant au moins 20 nucléotides, ainsi que les gènes ayant une homologie de séquence d'au moins 80 %.

5. Utilisation selon l'une des revendications 1 à 4, **caractérisée en ce que** les profils d'expression génique sont construits par des procédés d'hybridation, en particulier ceux qui sont à base de biopuces ou d'une PCR en temps réel.

6. Procédé pour la mesure in vitro de profils d'expression génique et/ou d'au moins une batterie d'activités géniques pour détecter une péritonite et une pneumonie chez des patients septiques, **caractérisé en ce qu'**on détermine dans un échantillon de sang d'un patient l'activité génique d'un grand nombre de gènes définis, associés à la péritonite et la pneumonie dans le cas d'une sepsie dans un échantillon de sang d'un patient, les gènes étant choisis dans le groupe consistant en SEQ ID NO:1 à SEQ ID NO:191, les fragments géniques de ces dernières ayant au moins 20 nucléotides, ainsi que les gènes ayant une homologie de séquence d'au moins 80 %, et étant regroupés en les batteries diagnostiques suivantes :
Batterie 1 : SEQ ID NO:1 à SEQ ID NO:77
Batterie 2 : SEQ ID NO:78 à SEQ ID NO:191
les polynucléotides SEQ ID 1 à 77 étant spécifiques de la péritonite, les polynucléotides SEQ ID 78 à SEQ ID NO:191 étant spécifiques de la pneumonie,
les séquences SEQ ID 1-4, 7-9, 11, 14-16, 21, 23, 26-29, 32, 34-38, 41-49, 51, 52, 54-63, 65, 67, 69-71, 73-75, 77 étant surexprimées dans le cas d'une péritonite, et les séquences SEQ ID 5, 6, 10, 12, 13, 17-20, 22, 24, 25, 30, 31, 33, 39, 40, 50, 53, 64, 66, 68, 72, 76 étant sous-exprimés dans le cas d'une péritonite, et
les séquences SEQ ID 80, 85-87, 91, 94, 97, 98, 100, 107, 111, 112, 117, 118, 120, 121, 123, 124, 127-129, 131, 133-135, 138, 142, 145, 149, 151-154, 156, 169, 162, 164, 165, 175, 182, 184, 186, 187, 191 étant surexprimées dans le cas d'une pneumonie, et les séquence SEQ ID 78, 79, 81-84, 88-90, 92, 93, 95, 96, 99, 101-106, 108-110, 113-116, 119, 122, 125, 126, 130, 132, 136, 137, 139-141, 143, 144, 146-148, 150, 155, 157, 158, 160, 161, 163, 167-174, 176-181, 183, 185, 188-190 étant sous-exprimées dans le cas d'une pneumonie.

7. Procédé selon la revendication 7, **caractérisé en ce que** les gènes ou fragments géniques listés dans la revendication 6 et/ou les séquences qui dérivent de leur ARN sont remplacés par des analogues synthétiques, des aptamères, des Spiegelmères, ainsi que des acides peptido- et morpholinonucléiques.

8. Procédé selon la revendication 6 ou 7, **caractérisé en ce que** les activités géniques sont déterminées par des procédés d'hybridation et/ou à l'aide de biopuces, et/ou sont déterminées par des procédés indépendants d'une hybridation, en particulier une hydrolyse enzymatique et/ou chimique et/ou des procédés d'amplification, de préférence une PCR, suivis d'une quantification des acides nucléiques et/ou de dérivés et/ou de fragments de ces derniers.

9. Utilisation de profils d'expression génique obtenus à partir d'un échantillon de sang d'un patient et/ou des sondes utilisées à cette fin, qui sont choisis dans le groupe consistant en les séquences SEQ ID NO:1 à SEQ ID NO:191 ainsi que les fragments géniques de ces dernières ayant au moins 20 nucléotides, pour déterminer l'activité génique, et/ou des produits protéiques qui en dérivent, pour le criblage de principes actifs contre la péritonite et/ou la pneumonie et/ou pour évaluer les effets thérapeutiques de principes actifs contre la péritonite et/ou la pneumonie.

10. Utilisation selon la revendication 9, **caractérisée en ce qu'**on utilise des analogues synthétiques pouvant subir une hybridation des sondes listées dans la revendication 8.

11. Trousse contenant un choix de séquences selon SEQ ID NO:1 à SEQ ID N0:191, qui sont spécifiques de la détection d'une péritonite et d'une pneumonie en présence d'une sepsie, et/ou de fragments géniques de ces dernières ayant au moins 20 nucléotides, pour déterminer les profils d'expression génique in vitro d'un échantillon de sang d'un patient, pour détecter une péritonite et une pneumonie chez des patients septiques.
